# EUROPÄISCHE PATENTSCHRIFT

(11) **EP 4 616 933 B1**
(45) Veröffentlichungstag und Bekanntmachung des Hinweises auf die Patenterteilung: **01.07.2026**
(21) Anmeldenummer: 24163708.1
(22) Anmeldetag: 15.03.2024
(51) Int. Cl.: B01F 23/50, B01F 35/60, B01F 35/71, B01F 101/20, A61B 17/88, B01F 31/40, B01F 33/501, B01F 35/75

(54) **VORRICHTUNG UND VERFAHREN ZUM BEREITSTELLEN EINES KNOCHENZEMENTTEIGS**
DEVICE AND METHOD FOR PROVIDING A BONE CEMENT DOUGH
DISPOSITIF ET PROCÉDÉ DE PRÉPARATION D'UNE PÂTE DE CIMENT OSSEUX

(43) Veröffentlichungstag der Anmeldung: 17.09.2025
(73) Patentinhaber: Heraeus Medical GmbH, 61273 Wehrheim (DE)
(72) Erfinder: Dr. Vogt, Sebastian, 61273 Wehrheim (DE); Dr. Kluge, Thomas, 61273 Wehrheim (DE)
(74) Vertreter: Heraeus IP

(56) Entgegenhaltungen:
- EP-A1- 3 231 505
- US-A1- 2016 354 129

## Beschreibung

Die Erfindung betrifft eine Vorrichtung zum Bereitstellen eines Knochenzementteigs aus zwei Ausgangskomponenten, umfassend
eine Mischeinheit mit einem Innenraum, in welchem ein Knochenzementpulver als erste Ausgangskomponente lagert oder lagerbar ist,
ein Reservoir mit einem Reservoirbehälter, in dem mindestens ein fluidleitend geschlossener Monomerflüssigkeitsbehälter gefüllt mit einer Monomerflüssigkeit als zweite Ausgangskomponente lagert oder lagerbar ist,
eine Transfereinheit umfassend einen Hohlzylinder und einen axial im Hohlzylinder verschiebbaren Förderkolben,
wobei die Mischeinheit und das Reservoir über den Hohlzylinder fluidleitend miteinander verbunden oder verbindbar sind,
wobei der Monomerflüssigkeitsbehälter in der Vorrichtung derart zu öffnen ist, dass die Monomerflüssigkeit aus dem Reservoir in den Hohlzylinder über eine Durchführung fließbar ist und aus dem Hohlzylinder in den Innenraum durch ein axiales Verschieben des Förderkolbens im Hohlzylinder förderbar ist.

Die Erfindung betrifft weiterhin ein Verfahren zum Bereitstellen eines Knochenzementteigs aus zwei Ausgangskomponenten mittels einer derartigen Vorrichtung.

### Hintergrund der Erfindung

Es werden erhebliche Bemühungen unternommen, Vorrichtungen und Verfahren zum Bereitstellen von Knochenzement aufzuzeigen, mittels derer Knochenzementteig einfach, zuverlässig und schnell bereitgestellt werden kann. Ein wichtiger Aspekt bei der Bereitstellung von Knochenzementteig ist die Vermeidung von Lufteinschlüssen, wie beispielsweise Gasblasen, im Knochenzement. Zu deren Vermeidung wurden eine Vielzahl von Vakuum-Zementiersystemen beschreiben, von denen exemplarisch folgende genannt sind: US 6,033,105 A, US 5,624,184 A, US 4,671,263 A, US 4,973,168 A, US 5,100,241 A, WO 99/67015 A1, EP 1020167 A2, US 5,586,821 A, EP 1016452 A2, DE 3640279 A1, WO 94/26403 A1, EP 1005901 A2, EP 1886647 A1, US 5,344,232 A.

Es besteht im Markt der Wunsch zur Vereinfachung der Bereitstellung von Knochenzementteig. Eine Weiterentwicklung besteht in der Entwicklung von Zementiersystemen, in denen beide Ausgangskomponenten in separaten Bereichen der Mischsysteme gelagert sind und erst unmittelbar vor der Zementierapplikation im Zementiersystem miteinander vermischt werden. Solche geschlossenen, sogenannten Full-Prepacked-Systeme, sind beispielsweise in folgenden Schriften genannt: EP 0 692 229 A1, DE 10 2009 031 178 B3, US 5,997,544 A, US 6,709,149 B1, DE 698 12 726 T2, EP 0 796 653 A2, US 5,588,745 A.

In den vorgenannten Full-Prepacked-Systemen erfolgt die Vermischung einer Monomerflüssigkeit mit einem Knochenzementpulver durch mechanisches Vermischen, beispielsweise mittels eines Mischstabes.

In der Patentanmeldung EP 3 093 067A1 wird eine weitere Vorrichtung zum Bereitstellen eines Knochenzementteigs beschrieben. Die Vorrichtung umfasst eine Mischeinheit, in der ein Knochenzementpulver als erste Ausgangskomponente und ein Reservoir, in dem eine Monomerflüssigkeit als zweite Ausgangskomponente in einer Ampulle lagert. Die Mischeinheit ist mit dem Reservoir fluidleitend über einen Hohlzylinder einer Transfereinheit verbunden. Nach einem fluidleitenden Öffnen der Ampulle durch ein Abknicken des Reservoirs, ist die Monomerflüssigkeit in den Hohlzylinder fließbar, von wo sie durch ein axiales Verschieben eines Förderkolbens der Transfereinheit im Hohlzylinder in die Mischeinheit förderbar ist. Nachteilig gestaltet sich bei einem derartigen System, dass die Schritte des fluidleitenden Öffnens und des Förderns der Monomerflüssigkeit aus dem Hohlzylinder in die Mischeinheit gleichzeitig stattfinden können. Dies hat zur Folge, dass beispielsweise das Fördern bereits ausgelöst wird, bevor alle Monomerflüssigkeit aus dem Reservoir in den Hohlzylinder geflossen ist, was zu unerwünschten Zusammensetzungen des Knochenzementteigs, beispielsweise durch unvollständiges Fördern der Monomerflüssigkeit, führt. Die Vorrichtung ist daher anfällig für Bedienungsfehler.

Es besteht daher im Markt der Wunsch zur weiteren Vereinfachung von Vorrichtungen zum Bereitstellen von Knochenzementteig und insbesondere für anwendungssichere Vorrichtungen, welche Bedienungsfehler möglichst ausschließen.

EP3231505A1 offenbart den Oberbegriff des Anspruchs 1.

### Aufgaben

Eine Aufgabe der vorliegenden Erfindung ist es, einen oder mehrere der sich aus dem Stand der Technik ergebenen Nachteile zumindest teilweise zu überwinden.

Im Speziellen basiert die Erfindung auf dem Ziel, eine Vorrichtung zur Verfügung zu stellen, welche ein einfaches und anwendungssicheres Bereitstellen eines Knochenzementteigs ermöglicht. Insbesondere soll die Vorrichtung ermöglichen, eine Monomerflüssigkeit möglichst anwendungssicher in der gewünschten Menge in ein Knochenzementpulver zu fördern, so dass ein Knochenzementteig mit einem gewünschten Mischungsverhältnis dieser beiden Ausgangskomponenten bereitstellbar ist. Insbesondere soll das Öffnen eines Monomerflüssigkeitsbehälter befüllt mit der Monomerflüssigkeit mit möglichst wenig Aufwand und unter Vermeidung zusätzlicher, separater Werkzeuge erfolgen. Weiterhin soll das Öffnen des Monomerflüssigkeitsbehälters mit möglichst wenigen Bauteilen ermöglicht sein. Weiterhin soll die Monomerflüssigkeit möglichst verlustfrei und schnell zum Bereitstellen des Knochenzementteigs zur Verfügung stehen. Das Fördern der Monomerflüssigkeit in ein Knochenzementpulver zur Bereitstellung des Knochenzementteigs soll mit möglichst geringem Kraftaufwand durchführbar sein. Die Vorrichtung soll mit möglichst einfachen Arbeitsschritten und möglichst anwendungssicher bedienbar sein.

Es ist eine weitere Aufgabe der Erfindung, ein Verfahren bereitzustellen, mit dem Knochenzement aus zwei Ausgangskomponenten bereitgestellt werden kann, mittels dem mindestens ein Teil der bereits beschriebenen Aufgaben zumindest zum Teil gelöst wird.

### Bevorzugte Ausführungsformen der Erfindung

Ein Beitrag zur mindestens teilweisen Erfüllung mindestens einer der zuvor genannten Aufgaben wird durch die Merkmale der unabhängigen Ansprüche geleistet. Die abhängigen Ansprüche stellen bevorzugte Ausführungsformen bereit, die zur mindestens teilweisen Erfüllung mindestens einer der Aufgaben beitragen.

Eine erste Ausführungsform der Erfindung ist eine Vorrichtung zum Bereitstellen eines Knochenzementteigs aus zwei Ausgangskomponenten, umfassend
eine Mischeinheit mit einem Innenraum, in welchem ein Knochenzementpulver als erste Ausgangskomponente lagert oder lagerbar ist, vorzugsweise lagert,
ein Reservoir mit einem Reservoirbehälter, in dem mindestens ein fluidleitend geschlossener Monomerflüssigkeitsbehälter gefüllt mit einer Monomerflüssigkeit als zweite Ausgangskomponente lagert oder lagerbar ist, vorzugsweise lagert,
eine Transfereinheit umfassend einen Hohlzylinder und einen axial im Hohlzylinder verschiebbaren Förderkolben,
wobei die Mischeinheit und das Reservoir über den Hohlzylinder fluidleitend miteinander verbunden oder verbindbar sind,
wobei der Monomerflüssigkeitsbehälter in der Vorrichtung derart zu öffnen ist, dass die Monomerflüssigkeit aus dem Reservoir in den Hohlzylinder über eine Durchführung fließbar ist und aus dem Hohlzylinder in den Innenraum durch ein axiales Verschieben des Förderkolbens im Hohlzylinder förderbar ist,
dadurch gekennzeichnet, dass
in einer ersten Konfiguration der Vorrichtung der Förderkolben mit einem Öffnungsmittel verbunden ist, so dass ein axiales Verschieben des Förderkolbens aus einer ersten Position in eine zweite Position das Öffnungsmittel gegen den Monomerflüssigkeitsbehälter schiebt, so dass der Monomerflüssigkeitsbehälter durch das Öffnungsmittel fluidleitend geöffnet werden kann und die Monomerflüssigkeit aus dem Reservoir über die Durchführung in den Hohlzylinder fließen kann, und dass
in einer zweiten Konfiguration der Vorrichtung der Förderkolben getrennt vom Öffnungsmittel ist, so dass die Monomerflüssigkeit durch ein fortgeführtes axiales Verschieben des Förderkolbens aus der zweiten Position in Richtung einer dritten Position aus dem Hohlzylinder in den Innenraum förderbar ist.

In einer Ausführungsform der Vorrichtung ist in der ersten Konfiguration der Förderkolben lediglich zwischen der ersten Position und der zweiten Position verschiebbar. Diese Ausführungsform ist eine zweite Ausführungsform der Erfindung, welche vorzugsweise von der ersten Ausführungsform der Erfindung abhängt.

In einer Ausführungsform der Vorrichtung liegt in der ersten Konfiguration das Öffnungsmittel in der zweiten Position des Förderkolbens an einem Anschlag an, so dass das fortgeführte axiale Verschieben des Förderkolbens aus der zweiten Position in Richtung der dritten Position im unterbunden ist. Diese Ausführungsform ist eine dritte Ausführungsform der Erfindung, welche vorzugsweise von der zweiten Ausführungsform der Erfindung abhängt.

In einer Ausführungsform der Vorrichtung ist das Öffnungsmittel ein Keil. Diese Ausführungsform ist eine vierte Ausführungsform der Erfindung, welche vorzugsweise von einer der vorhergehenden Ausführungsformen der Erfindung abhängt.

In einer Ausführungsform der Vorrichtung weist der Keil mindestens eine Durchführung auf, so dass die Monomerflüssigkeit durch die Durchbrechung und die Durchführung aus dem Reservoir in den Hohlzylinder fließbar ist. Diese Ausführungsform ist eine fünfte Ausführungsform der Erfindung, welche vorzugsweise von der vierten Ausführungsform der Erfindung abhängt.

In einer Ausführungsform der Vorrichtung, wobei der Monomerflüssigkeitsbehälter eine Ampulle, vorzugsweise eine Glasampulle, ist und im Reservoirbehälter lagert, wobei das Öffnungsmittel axial über einem Ampullenkopf der Ampulle angeordnet ist, so dass beim Verschieben des Förderkolbens aus der ersten Position in die zweite Position in der ersten Konfiguration der Vorrichtung das Öffnungsmittel gegen den Ampullenkopf pressbar oder schiebbar und so die Ampulle fluidleitend öffenbar ist. Diese Ausführungsform ist eine sechste Ausführungsform der Vorrichtung, welche vorzugsweise von einer der vorhergehenden Ausführungsformen der Erfindung abhängt.

In einer Ausführungsform der Vorrichtung sind der Förderkolben und das Öffnungsmittel in der ersten Konfiguration über einen Mitnehmer miteinander verbunden. Diese Ausführungsform ist eine siebte Ausführungsform der Vorrichtung, welche vorzugsweise von einer der vorhergehenden Ausführungsformen der Erfindung abhängt.

In einer Ausführungsform der Vorrichtung wird der Mitnehmer beim Verbringen der Vorrichtung aus der ersten Konfiguration in die zweite Konfiguration vom Öffnungsmittel getrennt. Diese Ausführungsform ist eine achte Ausführungsform der Vorrichtung, welche vorzugsweise von der siebten Ausführungsform der Erfindung abhängt.

In einer Ausführungsform der Vorrichtung ist das, vorzugsweise reversible, Verbringen der Vorrichtung aus der ersten Konfiguration in die zweite Konfiguration durch ein Drehen des Förderkolbens um eine Förderkolbenlängsachse erreichbar. Diese Ausführungsform ist eine neunte Ausführungsform der Erfindung, welche vorzugsweise von der achten Ausführungsform der Erfindung abhängt.

In einer Ausführungsform der Vorrichtung ist der Mitnehmer an einem Ring um den Förderkolben angeordnet, so dass das Verbringen der Vorrichtung aus der ersten Konfiguration in die zweite Konfiguration durch ein Drehen des Ringes um den Förderkolben erreichbar ist. Diese Ausführungsform ist eine zehnte Ausführungsform der Erfindung, welche vorzugsweise von der achten Ausführungsform der Erfindung abhängt.

In einer Ausführungsform der Vorrichtung weist die Vorrichtung eine Transportsicherung auf, welche ein unbeabsichtigtes Verschieben des Öffnungsmittels verhindert. Diese Ausführungsform ist eine elfte Ausführungsform der Erfindung, welche vorzugsweise von einer der vorhergehenden Ausführungsformen der Erfindung abhängt.

In einer Ausführungsform der Vorrichtung weist der Förderkolben mindestens ein lösbares, vorzugsweise reversibel lösbares, Kontrollelement auf, welches das Verschieben des Förderkolbens zwischen der zweiten Position und der dritten Position auf eine Zwischenposition, insbesondere auf eine Zwischenposition räumlich zwischen der zweiten Position und dritten Position, begrenzt. Diese Ausführungsform ist eine zwölfte Ausführungsform der Erfindung, welche vorzugsweise von einer der vorhergehenden Ausführungsformen der Erfindung abhängt.

In einer Ausführungsform der Vorrichtung verschließt der Förderkolben beim axialen Verschieben aus der zweiten Position in Richtung der dritten Position die Durchführung fluidleitend. Diese Ausführungsform ist eine dreizehnte Ausführungsform der Erfindung, welche vorzugsweise von einer der vorhergehenden Ausführungsformen der Erfindung abhängt.

Eine vierzehnte Ausführungsform der Erfindung ist ein Verfahren zur Bereitstellung eines Knochenzementteigs aus zwei Ausgangskomponenten mittels einer Vorrichtung nach einer der Ausführungsformen eins bis dreizehn der Erfindung, wobei im Innenraum ein Knochenzementpulver als erste Ausgangskomponente und im Reservoirbehälter ein fluidleitend geschlossener Monomerflüssigkeitsbehälter gefüllt mit einer Monomerflüssigkeit als zweite Ausgangskomponente lagert, umfassend die Verfahrensschritte:
a. Bereitstellen der Vorrichtung in der ersten Konfiguration;
b. Axiales Verschieben des Förderkolbens aus der ersten Position in die zweite Position unter fluidleitendem Öffnen des Monomerflüssigkeitsbehälters;
c. Fließen der Monomerflüssigkeit aus dem Reservoir in den Hohlzylinder;
d. Verbringen der Vorrichtung aus der ersten Konfiguration in die zweite Konfiguration;
e. Axiales Verschieben des Förderkolbens aus der zweiten Position in Richtung der dritten Position unter Fördern der Monomerflüssigkeit aus dem Hohlzylinder in den Innenraum;
f. Mischen des Knochenzementpulvers und der Monomerflüssigkeit in der Mischeinheit unter Bereitstellen des Knochenzementteigs.

In einer Ausführungsform des Verfahrens geschieht das Verbringen der Vorrichtung aus der ersten Konfiguration in die zweite Konfiguration durch eine Drehbewegung. Diese Ausführungsform ist eine fünfzehnte Ausführungsform der Erfindung, welche vorzugsweise von der vierzehnten Ausführungsform der Erfindung abhängt.

### Allgemeines

In der vorliegenden Beschreibung beinhalten Bereichsangaben auch die als Grenzen genannten Werte. Eine Angabe der Art "im Bereich von X bis Y" in Bezug auf eine Größe A bedeutet folglich, dass A die Werte X, Y und Werte zwischen X und Y annehmen kann. Einseitig begrenzte Bereiche der Art "bis zu Y" für eine Größe A bedeuten entsprechend als Wert Y und kleiner als Y.

Einige der beschriebenen Merkmale sind mit dem Begriff "im Wesentlichen" verknüpft. Der Begriff "im Wesentlichen" ist so zu verstehen, dass unter realen Bedingungen und Fertigungstechniken eine mathematisch exakte Auslegung von Begrifflichkeiten wie "Überlagerung", "senkrecht", "Durchmesser" oder "Parallelität" nie exakt, sondern nur innerhalb gewisser fertigungstechnischer Fehlertoleranzen gegeben sein kann. Beispielsweise schließen "im Wesentlichen senkrechte Achsen" einen Winkel von 85 Grad bis 95 Grad zueinander ein und "im Wesentlichen gleiche Volumen" umfassen eine Abweichung von bis zu 5 Volumen-%. Eine "im Wesentlichen aus Kunststoff bestehende Vorrichtung" umfasst beispielsweise einen Kunststoffanteil von ≥95 bis ≤100 Gewichts-%. Eine "im Wesentlichen vollständige Befüllung eines Volumens B" umfasst beispielsweise eine Befüllung von ≥95 bis ≤100 Volumen-% des Gesamtvolumens von B.

Die Begriffe "proximal" und "distal" dienen lediglich der Bezeichnung der räumlich gegenüberliegenden Enden der Vorrichtung oder anderer Struktureinheiten der Vorrichtung und erlauben keinen Rückschluss auf die Orientierung in Bezug zu einem menschlichen Körper, beispielsweise eines Anwenders der Vorrichtung. "Distal zu ..." und "proximal zu..." oder ähnliche Formulierungen drücken entsprechend lediglich die räumliche Anordnung zweier Struktureinheiten der Vorrichtung zueinander aus.

### Ausführliche Beschreibung

Ein erster Gegenstand der Erfindung betrifft eine Vorrichtung zum Bereitstellen eines Knochenzementteigs aus zwei Ausgangskomponenten, umfassend
eine Mischeinheit mit einem Innenraum, in welchem ein Knochenzementpulver als erste Ausgangskomponente lagert oder lagerbar ist,
ein Reservoir mit einem Reservoirbehälter, in dem mindestens ein fluidleitend geschlossener Monomerflüssigkeitsbehälter gefüllt mit einer Monomerflüssigkeit als zweite Ausgangskomponente lagert oder lagerbar ist,
eine Transfereinheit umfassend einen Hohlzylinder und einen axial im Hohlzylinder verschiebbaren Förderkolben,
wobei die Mischeinheit und das Reservoir über den Hohlzylinder fluidleitend miteinander verbunden oder verbindbar sind,
wobei der Monomerflüssigkeitsbehälter in der Vorrichtung derart zu öffnen ist, dass die Monomerflüssigkeit aus dem Reservoir in den Hohlzylinder über eine Durchführung fließbar ist und aus dem Hohlzylinder in den Innenraum durch ein axiales Verschieben des Förderkolbens im Hohlzylinder förderbar ist,
dadurch gekennzeichnet, dass
in einer ersten Konfiguration der Vorrichtung der Förderkolben mit einem Öffnungsmittel verbunden ist, so dass ein axiales Verschieben des Förderkolbens aus einer ersten Position in eine zweite Position das Öffnungsmittel gegen den Monomerflüssigkeitsbehälter schiebt, so dass der Monomerflüssigkeitsbehälter durch das Öffnungsmittel fluidleitend geöffnet werden kann und die Monomerflüssigkeit aus dem Reservoir über die Durchführung in den Hohlzylinder fließen kann, und dass
in einer zweiten Konfiguration der Vorrichtung der Förderkolben getrennt vom Öffnungsmittel ist, so dass die Monomerflüssigkeit durch ein fortgeführtes axiales Verschieben des Förderkolbens aus der zweiten Position in Richtung einer dritten Position aus dem Hohlzylinder in den Innenraum förderbar ist.

Vorzugsweise lagert sowohl das Knochenzementpulver als auch mindestens ein Monomerflüssigkeitsbehälter in der Vorrichtung.

Die Vorrichtung dient einem Anmischen eines Knochenzementteigs aus einem Knochenzementpulver und einer Monomerflüssigkeit, wobei vor dem Anmischen das Knochenzementpulver in einer Mischeinheit der Vorrichtung lagerbar ist oder, bevorzugt, lagert und die Monomerflüssigkeit in einem Reservoir der Vorrichtung lagerbar ist oder, bevorzugt, lagert. Vorzugsweise befindet sich die Monomerflüssigkeit im Reservoir, insbesondere in einem Reservoirbehälter des Reservoirs, innerhalb eines oder mehrerer Monomerflüssigkeitsbehälter. Der Reservoirbehälter ist dazu ausgelegt, diesen oder diese Monomerflüssigkeitsbehälter sicher und steril zu lagern.

Ein Monomerflüssigkeitsbehälter ist ein Gefäß für die Monomerflüssigkeit, in dem diese sicher und insbesondere steril bis zu ihrer Verwendung lagerbar ist. Dem Fachmann sind unterschiedliche Monomerflüssigkeitsbehälter bekannt.

Beispielsweise kann der Monomerflüssigkeitsbehälter ein Beutel sein. Bevorzugte Beutel sind Mehrschichtverbundfolien mit einer EVOH-Sperrschicht (Ethylen-Vinylalkohol-Sperrschicht), optional umfassend eine Metallbeschichtung, insbesondere umfassend eine Aluminiumbeschichtung.

Vorzugsweise ist der Monomerflüssigkeitsbehälter eine Ampulle, vorzugsweise eine Glasampulle. Ampullen sind bevorzugt, da diese kontrollierbarer zu öffnen sind und ein vollständiges Ausfließen der Monomerflüssigkeit aus fluidleitend geöffneten Ampullen erleichtert ist.

Die Mischeinheit dient einem Anmischen des Knochenzementteigs aus dem Knochenzementpulver und der Monomerflüssigkeit nach einem Fördern der Monomerflüssigkeit in die Mischeinheit, insbesondere nach einem Fördern der Monomerflüssigkeit in einen Innenraum der Mischeinheit, in welchem vorzugsweise das Knochenzementpulver lagert.

Die Mischeinheit weist vorzugsweise eine hohlzylinderförmige Kartusche auf. Unter einer hohlzylinderförmigen Kartusche ist ein rohrartiges Behältnis zu verstehen, welches einen Innenraum und eine den Innenraum umgebende Kartuschenwand aufweist. Der Querschnitt der Kartusche kann beliebige Formen annehmen. Aufgrund der einfachen Fertigung und der anwendungssichereren Verwendung der Vorrichtung ist der Querschnitt, und bevorzugt auch der Querschnitt des Innenraums, kreisförmig ausgestaltet. Dies erlaubt eine gute Handhabbarkeit für den Anwender und reduziert durch eine Abwesenheit von Kanten ein Risiko einer Verkeilung beweglicher Teile innerhalb der Vorrichtung. Erfindungsgemäß kann die Kartusche aus unterschiedlichsten Materialien oder Materialkombinationen bestehen. Beispiele kann die Vorrichtung aus einem Polymer bestehen. Bevorzugt handelt es sich bei dem Polymer um ein transparentes Polymer, da der Anwender auf diese Weise eine ordnungsgemäße Funktion der Vorrichtung während einer Verwendung optisch kontrollieren kann.

Um den Knochenzementteig aus den beiden Ausgangsmaterialien in der Mischeinheit anzumischen, ist im Innenraum vorzugsweise ein axial im Innenraum reversibel verschiebbares Mischelement angeordnet. Ein reversibles Hin- und Herschieben des Mischelements im Innenraum führt zu einem möglichst homogenen Durchmischen des Knochenzementpulvers und der Monomerflüssigkeit unter Bereitstellung des Knochenzementteigs. Beispielsweise kann das Mischelement eine Mischstange sein, wobei an einem in Innenraum angeordneten Ende der Mischstange rotorartige Mischflügel angeordnet sind. Vorzugsweise ist die Mischstange auch um ihre Längsachse drehbar im Innenraum gelagert, so dass ein derartiges Drehen die Mischflügel den Mischvorgang der beiden Ausgangskomponenten unterstützen kann.

Die Mischeinheit weist vorzugsweise eine Austragsöffnung auf, aus welcher der in der Mischeinheit bereitgestellte Knochenzementteigs austragbar ist. Vorzugsweise ist die Mischeinheit über die Austragsöffnung fluidleitend mit der Transfereinheit, vorzugsweise über eine Verbindungsleitung, wie beispielsweise einen Schlauch, verbunden. Zum Austragen des Knochenzementteigs aus der Mischeinheit ist es somit bevorzugt, die Mischeinheit von der restlichen Vorrichtung, insbesondere der Transfereinheit und auch dem Schlauch zwischen der Mischeinheit und der Transfereinheit, zu lösen, so dass der Knochenzementteig durch die Austragsöffnung austragbar ist.

Zum Austragen des Knochenzementteigs weist die Mischeinheit vorzugsweise einen axial im Innenraum beweglichen Förderkolben auf.

Fluidleitend zwischen dem Reservoir und der Mischeinheit ist die Transfereinheit angeordnet. Die Transfereinheit weist einen Hohlzylinder und einen im Hohlzylinder axial verschiebbaren Förderkolben auf. Der Hohlzylinder ist fluidleitend über eine Durchführung mit dem Reservoir, insbesondere dem Reservoirbehälter, verbunden, so dass die Monomerflüssigkeit eines im Reservoir fluidleitend geöffneter Monomerflüssigkeitsbehälters über die Durchführung in den Hohlzylinder fließen kann. Der Hohlzylinder ist dazu ausgelegt, die im Reservoir bei Benutzung der Vorrichtung lagernde Monomerflüssigkeit vollständig aufzunehmen. Zwischen Hohlzylinder und Mischeinheit besteht ebenso eine fluidleitende Verbindung, so dass die sich im Hohlzylinder befindliche Monomerflüssigkeit in die Mischeinheit transferierbar ist. Vorzugsweise ist der Hohlzylinder mit der Mischeinheit über eine Verbindungsleiten, vorzugsweise einen Schlauch fluidleitend verbunden. Vorzugsweise ist die Verbindungsleitung zwischen der Transfereinheit und der Mischeinheit in einer Schlaufe mit einem hohen Scheitelpunkt angeordnet, um ein unkontrolliertes Durchfließen der Monomerflüssigkeit durch die Verbindungsleitung in die Mischeinheit, insbesondere den Innenraum, zu verhindern.

Die im Hohlzylinder befindliche Monomerflüssigkeit verbleibt somit vorzugsweise innerhalb des Hohlzylinders, bis ein Anwender der Vorrichtung aktiv eingreift, um die Monomerflüssigkeit in die Mischeinheit, insbesondere den Innenraum, zu fördern.

Um die Monomerflüssigkeit aus dem Hohlzylinder in die Mischeinheit, insbesondere den Innenraum, zu fördern, weist die Transfereinheit einen Förderkolben auf. Der Förderkolben ist axial im Hohlzylinder verschiebbar und bildet mit dem Hohlzylinder ein Kolben-Zylinder-System aus zum Fördern der Monomerflüssigkeit aus. Vorzugsweise befindet sich die Verbindungsleitung an einem axialen Ende des Hohlzylinders, so dass ein Verschieben des Förderkolbens in Richtung der Verbindungsleitung ein, im Wesentlichen vollständiges, Fördern der Monomerflüssigkeit aus dem Hohlzylinder in die Mischeinheit bewirken kann.

Das Reservoir, insbesondere der Reservoirbehälter, dient dem Lagern des mit der Monomerflüssigkeit gefüllten Monomerflüssigkeitsbehälters, wobei der Monomerflüssigkeitsbehälter bis zu seiner Verwendung fluidleitend geschlossen bleibt. Um den fluidleitend geschlossenen Monomerflüssigkeitsbehälter fluidleitend zu öffnen, weist die Vorrichtung ein Öffnungsmittel auf. Das Öffnungsmittel ist auf die Art und Beschaffenheit des Monomerflüssigkeitsbehälters ausgelegt, ist aber so beschaffen, dass ein Schieben, oder in anderen Worten "Pressen", des Öffnungsmittels gegen den Monomerflüssigkeitsbehälters die strukturelle Integrität desselben überwindet und so fluidleitend öffnet. Dazu weist das Öffnungsmittel vorzugsweise eine spitze oder scharfkantige Seite oder Fläche auf, welche gegen den Monomerflüssigkeitsbehälter schiebbar gelagert ist. Vorzugsweise ist das Öffnungsmittel innerhalb des Reservoirs angeordnet, so dass es in Kontakt mit dem Monomerflüssigkeitsbehälter verschiebbar ist.

In einer ersten Konfiguration der Vorrichtung ist der Förderkolben mit dem Öffnungsmittel derart verbunden, dass ein axiales Verschieben des Förderkolbens aus einer ersten Position, insbesondere aus einer ersten räumlichen Position innerhalb des Hohlzylinders, in eine zweite Position, insbesondere in eine zweite räumliche Position innerhalb des Hohlzylinders, das Öffnungsmittel gegen den Monomerflüssigkeitsbehälter schiebt und diesen somit fluidleitend öffnen kann. Dies bedeutet, dass sich in der ersten Konfiguration eine Bewegung des Förderkolbens auf den Förderkolben überträgt. In anderen Worten ist das Öffnungsmittel in der ersten Konfiguration über den Förderkolben bedienbar. Vorzugsweise führt ein axiales Verschieben des Förderkolbens innerhalb des Hohlzylinders zu einer gleichartigen Verschiebung des Öffnungsmittels innerhalb des Reservoirs, vorzugsweise um die gleiche Strecke.

In einer zweiten Konfiguration der Vorrichtung ist der Förderkolben getrennt vom Öffnungsmittel. Eine Bewegung, insbesondere ein axiales Verschieben des Förderkolbens innerhalb des Hohlzylinders führt somit im Gegensatz zur ersten Konfiguration nicht zu einem Verschieben des Öffnungsmittels.

In der zweiten Konfiguration führt ein fortgeführtes axiales Verschieben, also ein Verschieben in die gleiche Richtung, des Förderkolbens aus der zweiten Position in Richtung einer dritten Position, insbesondere in Richtung einer dritten räumlichen Position innerhalb des Hohlzylinders, zu einem Fördern der im Hohlzylinder befindlichen Monomerflüssigkeit in die Mischeinheit, insbesondere den Innenraum der Mischeinheit.

Die Vorrichtung weist somit zwei unterschiedliche Konfigurationen auf. Die erste Konfiguration dient dem fluidleitenden Öffnen des Monomerflüssigkeitsbehälters und damit verbunden einem Fließen der Monomerflüssigkeit aus dem Reservoir in den Hohlzylinder der Transfereinheit, während die zweite Konfiguration dem Fördern der Monomerflüssigkeit aus der Transfereinheit in die Mischeinheit dient. Die zwei Konfigurationen der Vorrichtung trennen somit funktionell und, durch den zeitlichen Verzug durch den Konfigurationswechsel, zeitlich die zwei Verfahrensschritte beim Bereitstellen des Knochenzementteigs, was die Anwendungssicherheit der Vorrichtung für einen Anwender erhöht. Insbesondere wird somit die Gefahr verringert, dass ein Fördern der Monomerflüssigkeit durch den Anwender ausgelöst wird, bevor diese im Wesentlichen vollständig aus dem Reservoir in die Transfereinheit geflossen ist.

Eine Ausführungsform der Vorrichtung ist dadurch gekennzeichnet, dass in der ersten Konfiguration der Förderkolben lediglich zwischen der ersten Position und der zweiten Position verschiebbar ist. Zum Fördern der Monomerflüssigkeit aus der Transfereinheit in die Mischeinheit muss die Vorrichtung in dieser Ausführungsform in der zweiten Konfiguration vorliegen, was die Anwendungssicherheit weiter erhöht. In der ersten Konfiguration ist hingegen lediglich ein fluidleitendes Öffnen des Monomerflüssigkeitsbehälters, aber kein Fördern der Monomerflüssigkeit aus der Transfereinheit in die Mischeinheit möglich.

Die Vorrichtung kann unterschiedlich ausgestaltet sein, dass das Fördern der Monomerflüssigkeit aus der Transfereinheit in die Mischeinheit lediglich in der zweiten Konfiguration, jedoch nicht in der ersten Konfiguration, ermöglicht ist.

Eine Ausführungsform der Vorrichtung ist dadurch gekennzeichnet, dass in der ersten Konfiguration das Öffnungsmittel in der zweiten Position des Förderkolbens an einem Anschlag anliegt, so dass das fortgeführte axiale Verschieben aus der zweiten Position in Richtung der dritten Position unterbunden ist. Der Anschlag verhindert ein fortgesetztes Verschieben des Öffnungsmittels. Da das Öffnungsmittel in der ersten Konfiguration mit dem Förderkolben verbunden ist, sorgt das blockierte Öffnungsmittel dafür, dass auch der Förderkolben nicht von der zweiten Position in Richtung der dritten Position verschiebbar ist. Da das Fördern der Monomerflüssigkeit erst durch ein Verschieben des Förderkolbens aus der zweiten Position in Richtung der dritten Position einsetzt, sorgt der Anschlag dafür, dass ein Fördern in der ersten Konfiguration der Vorrichtung nicht möglich ist. Dies erhöht die Anwendungssicherheit der Vorrichtung, da ein aktives Umschalten von der ersten Konfiguration in die zweite Konfiguration notwendig ist, um das Fördern der Monomerflüssigkeit in die Mischeinheit einzuleiten.

Der Anschlag kann an unterschiedlichen Stellen des Öffnungsmittels in der zweiten Position des Förderkolbens ansetzen, um den Förderkolben in Richtung der dritten Position zu blockieren. Beispielsweise kann der Anschlag eine Wand innerhalb des Reservoirs sein, gegen welche ein dem Monomerflüssigkeitsbehälter zugewandtes Ende des Öffnungsmittels, welches zum fluidleitenden Öffnen verwendet wurde, in der zweiten Position des Förderkolbens stößt. Der Anschlag kann auch ein Vorsprung innerhalb oder außerhalb des Reservoirs sein, gegen welche eine Ausstülpung am Öffnungsmittel in der zweiten Position des Förderkolbens stößt und dieses so blockiert. Diese Ausstülpung kann auch an einem dem Monomerflüssigkeitsbehälter abgewandten Ende des Öffnungsmittels angeordnet sein.

Das Öffnungsmittel kann unterschiedlich ausgestaltet sein, um den Monomerflüssigkeitsbehälter fluidleitend zu öffnen. Insbesondere richtet sich die Ausgestaltungsform des Öffnungsmittels nach der Art des Monomerflüssigkeitsbehälters. Beispielsweise kann das Öffnungsmittel ein Dorn sein, welcher insbesondere bei der Verwendung eines Beutels als Monomerflüssigkeitsbehälters bevorzugt ist.

Eine Ausführungsform der Vorrichtung ist dadurch gekennzeichnet, dass das Öffnungsmittel ein Keil ist. Der Keil weist zumindest in Richtung des Monomerflüssigkeitsbehälters, in diesem Fall bevorzugt einer Ampulle, mindestens eine, vorzugsweise scharfkantige, Kante auf, welche ein Öffnen des Monomerflüssigkeitsbehälters erleichtert.

Eine Ausführungsform der Vorrichtung ist dadurch gekennzeichnet, dass der Keil mindestens eine Durchbrechung, insbesondere fluidleitende Durchbrechung, aufweist, so dass die Monomerflüssigkeit durch die Durchbrechung und die Durchführung aus dem Reservoir in den Hohlzylinder fließbar ist. Je nach Ausgestaltung der Vorrichtung kann das Öffnungsmittel in Form eines Keils zeitlich nach dem Öffnen des Monomerflüssigkeitsbehälter ein Ausfließen der Monomerflüssigkeit aus dem Monomerflüssigkeitsbehälter, vorzugsweise in Form einer Ampulle, erschweren, da sich der Keil in der natürlichen Fließstrecke der Monomerflüssigkeit in Richtung der Durchführung, und somit in Richtung der Transfereinheit, befindet. Die mindestens eine Durchbrechung sorgt in einer derartigen Ausgestaltung für eine verbesserte Fließfähigkeit der Monomerflüssigkeit. Beispielsweise ist die Durchbrechung in Form eines Loches im Keil ausgeformt. In einer weiteren Ausgestaltung teilt die Durchbrechung eine Kante des Keils in zwei Kantenteilstücke, so dass die Monomerflüssigkeit zwischen diesen Kantenteilstücken durchfließbar ist.

Eine Ausführungsform der Vorrichtung ist dadurch gekennzeichnet, dass der Monomerflüssigkeitsbehälter eine Ampulle, vorzugsweise Glasampulle, ist und im Reservoirbehälter lagert und dass das Öffnungsmittel axial über einem Ampullenkopf der Ampulle angeordnet ist, so dass beim Verschieben des Förderkolbens aus der ersten Position in die zweite Position in der ersten Konfiguration der Vorrichtung das Öffnungsmittel gegen den Ampullenkopf pressbar und so die Ampulle fluidleitend öffenbar ist. In dieser Ausführungsform sind die Ampulle und das Öffnungsmittel derart zueinander ausgerichtet, dass das Öffnungsmittel bei der ordnungsgemäßen Verwendung gegen den Ampullenkopf der Ampulle drückt und die Ampulle im Bereich des Ampullenkopfes, vorzugsweise an einem Ampullenhals der Ampulle, fluidleitend öffnet. Bei den dem Fachmann bekannten Ampullen, insbesondere Glasampullen, für Monomerflüssigkeiten ist ein Ampullenkörper, welcher den Großteil der Monomerflüssigkeit aufzunehmen vermag, über einen schmaleren Ampullenhals mit dem Ampullenkopf verbunden. Der Ampullenhals stellt eine Sollbruchstelle der Ampulle dar, an welcher diese leicht und kontrolliert fluidleitend öffenbar ist. Üblicherweise wird dazu ein Druck gegen den Ampullenkopf ausgeübt, welcher zu einem Abbrechen desselben am Ampullenhals führt.

Der Förderkolben und das Öffnungsmittel können auf unterschiedliche Weise in der ersten Konfiguration der Vorrichtung miteinander verbunden sein, so dass eine Verschiebung des Förderkolbens eine Verschiebung des Öffnungsmittels bewirkt.

Eine Ausführungsform der Vorrichtung ist dadurch gekennzeichnet, dass der Förderkolben und das Öffnungsmittel in der ersten Konfiguration über einen Mitnehmer miteinander verbunden sind. Ein Mitnehmer ist eine Struktureinheit der Vorrichtung, welche eine direkte Verbindung zwischen Förderkolben und Öffnungsmittel herstellt. Der Mitnehmer führt somit zu einer synchronen, gleichlaufenden Verschiebung um einen gleichen Streckenbetrag von Förderkolben und Öffnungsmittel.

In dieser Ausführungsform kann ein Wechsel von der ersten Konfiguration in die zweite Konfiguration auf unterschiedliche Weise stattfinden. Beispielsweise kann der Mitnehmer sowohl vom Förderkolben als auch vom Öffnungsmittel gelöst werden, so dass der Förderkolben und das Öffnungsmittel nicht mehr miteinander verbunden sind. Oder der Mitnehmer wird vom Förderkolben gelöst, allerdings nicht vom Öffnungsmittel. Auch dies führt dazu, dass der Förderkolben und das Öffnungsmittel nicht mehr miteinander verbunden sind und eine Verschiebung des Förderkolbens sich nicht auf das Öffnungsmittel auswirkt.

Eine Ausführungsform der Vorrichtung ist dadurch gekennzeichnet, dass der Mitnehmer beim Verbringen der Vorrichtung aus der ersten Konfiguration in die zweite Konfiguration vom Öffnungsmittel getrennt, oder in anderen Worten gelöst, wird. In dieser Ausführungsform verbleibt der Mitnehmer in der zweiten Konfiguration vorzugsweise mit dem Förderkolben verbunden.

Das Trennen des Mitnehmers vom Öffnungsmittel beim Wechsel von der ersten Konfiguration in die zweite Konfiguration kann auf unterschiedliche Weise realisierbar sein. Beispielsweise kann der Mitnehmer vom Öffnungsmittel abziehbar ausgestaltet sein oder der Mitnehmer ist mit einer lösbaren Halterung ausgestaltet, welcher in der ersten Konfiguration geschlossen und in der zweiten Konfiguration geöffnet ist.

Eine Ausführungsform der Vorrichtung ist dadurch gekennzeichnet, dass das Verbringen der Vorrichtung aus der ersten Konfiguration in die zweite Konfiguration durch ein Drehen des Förderkolben um eine Förderkolbenlängsachse erreichbar ist. Das Drehen um die Förderkolbenlängsachse sorgt somit für ein Lösen des Mitnehmers vom Öffnungsmittel, wobei der Mitnehmer mit dem Förderkolben verbunden bleibt und sich gleichsam mit dem Förderkolben dreht. Der Mitnehmer ist in dieser Ausführungsform vorzugsweise in der ersten Konfiguration mit dem Öffnungsmittel derart in einem Eingriff stehend, dass eine axiale Verschiebung des Förderkolbens, und somit auch des Mitnehmers, sich auf das Öffnungsmittel überträgt, sich dieser Eingriff aber durch die Drehung des Förderkolbens, und somit auch des Mitnehmers, löst. Beispielsweise weist der Mitnehmer an einem dem Öffnungsmittel zugewandten Ende einen Stift auf, welcher in der ersten Konfiguration der Vorrichtung in eine stiftförmige Ausnehmung am Öffnungsmittel eingreift und so beide Elemente miteinander verbindet. Durch das Drehen des Förderkolbens kommt es zu einem Ausgleiten des Stiftes aus der Ausnehmung und der Mitnehmer und das Öffnungsmittel sind nicht mehr miteinander verbunden, so dass sich die Vorrichtung in der zweiten Konfiguration befindet.

Eine Ausführungsform der Vorrichtung ist dadurch gekennzeichnet, dass der Mitnehmer an einem Ring um den Förderkolben, insbesondere um einen Förderkolbenumfang des Förderkolbens, angeordnet ist, so dass das Verbringen der Vorrichtung aus der ersten Konfiguration in die zweite Konfiguration durch ein Drehen des Ringes um den Förderkolben erreichbar ist. Diese Ausführungsform ähnelt der vorhergehenden Ausführungsform, nur sorgt nicht ein Drehen des Förderkolbens, sondern das Drehen des Ringes für ein Lösen des Mitnehmers vom Öffnungsmittel. Der Mitnehmer verbleibt zwar auch in dieser Ausführungsform beim Förderkolben, allerdings ist der Mitnehmer unabhängig von einer etwaigen Drehbewegung des Förderkolbens für sich selbst drehbar. Der Ring, über welchen der Mitnehmer am Förderkolben angeordnet ist, kann sich vollständig oder auch nur teilweise um einen Förderkolbenumfang erstrecken. Beispielsweise kann der Ring eine Art Klemme sein, welcher den Mitnehmer drehbar am Förderkolben befestigt. Unabhängig von der genauen Ausgestaltung des Ringes, sorgt dieser aber immer dafür, dass eine axiale Verschiebung des Förderkolbens eine gleichartige Verschiebung des Mitnehmers zur Folge hat.

Eine Ausführungsform der Vorrichtung ist dadurch gekennzeichnet, dass die Vorrichtung eine Transportsicherung aufweist, welche ein unbeabsichtigtes Verschieben des Öffnungsmittel, insbesondere auch in der ersten Konfiguration der Vorrichtung, verhindert. Die Transportsicherung sorgt somit insbesondere für einen sicheren Transport der Vorrichtung, insbesondere falls der Monomerflüssigkeitsbehälter sich bereits im Reservoirbehälter befindet.

Zum Fördern der Monomerflüssigkeit aus dem Hohlzylinder in den Innenraum der Mischeinheit ist der Förderkolben in der zweiten Konfiguration der Vorrichtung aus der zweiten Position in Richtung der dritten Position verschiebbar. Das Fördern der Monomerflüssigkeit kann unmittelbar mit dem Beginn der Verschiebung des Förderkolbens aus der zweiten Position in Richtung der dritten Position einsetzen. Das Fördern der Monomerflüssigkeit kann auch erst einsetzen, nachdem der Förderkolben bereits ein Teilstück zwischen der zweiten Position und der dritten Position zurückgelegt hat. Beispielsweise falls dieses Teilstück zeitlich vor dem Verschieben des Förderkolbens nicht mit Monomerflüssigkeit, sondern mit Gas, beispielsweise Luft oder einem Schutzgas, wie beispielsweise Stickstoff, befüllt ist. Dies ist üblicherweise bedingt durch das Volumen des Hohlzylinders in Abhängigkeit der Verwendeten Menge der Monomerflüssigkeit.

Die dritte Position kann als die Position des Förderkolbens angesehen werden, welche ein im Wesentlichen vollständiges Fördern der Monomerflüssigkeit aus dem Hohlzylinder heraus ermöglicht. Ein Verschieben des Förderkolbens aus der zweiten Position in Richtung der dritten Position und letztendlich bis in die dritte Position ermöglicht somit ein anteiliges oder auch, bei Erreichen der dritten Position, ein im Wesentlichen vollständiges Fördern der Monomerflüssigkeit.

Beispielsweise kann es gewünscht sein, um ein bestimmtes Mischungsverhältnis von Knochenzementpulver zu Monomerflüssigkeit zu erhalten, dass nicht die gesamte im Monomerflüssigkeitsbehälter vorhandene Monomerflüssigkeit in die Mischeinheit gefördert werden soll. Unter diesen Umständen ist der Förderkolben nur ein entsprechendes Teilstück von der zweiten Position in die dritte Position zu verschieben, nicht allerdings die ganze Strecke bis zur dritten Position.

Eine Ausführungsform der Vorrichtung ist dadurch gekennzeichnet, dass der Förderkolben mindestens ein lösbares Kontrollelement aufweist, welches das Verschieben des Förderkolbens aus der zweiten Position und der dritten Position auf eine Zwischenposition begrenzt. Das Kontrollelement erlaubt es einem Anwender der Vorrichtung somit einzustellen, bis zu welcher Position der Förderkolben in der zweiten Konfiguration verschiebbar sein soll, was eine direkte Auswirkung auf die Menge der dabei geförderten Monomerflüssigkeit aus dem Hohlzylinder in den Innenraum zur Folge hat. Beispielsweise kann das Kontrollelement so eingestellt werden, dass der Förderkolben nur bis zu einer Zwischenposition in den Hohlzylinder einschiebbar ist, welche ein Fördern der Hälfte der im Hohlzylinder vorhandenen Menge an Monomerflüssigkeit erlaubt.

Das Kontrollelement kann beispielsweise eine Klammer, eine Manschette, eine Spannvorrichtung oder eine Klemme sein, welche an einer Außenseite des Förderkolbens lösbar befestigbar ist und so ein Verschieben des Förderkolbens in Richtung der dritten Position auf eine Zwischenposition begrenzt.

Ein weiterer Gegenstand der Erfindung betrifft ein Verfahren zur Bereitstellung eines Knochenzementteigs aus zwei Ausgangskomponenten mittels einer Vorrichtung nach einer der vorhergehenden Ausführungsformen, wobei im Innenraum ein Knochenzementpulver als erste Ausgangskomponente und im Reservoirbehälter ein fluidleitend geschlossener Monomerflüssigkeitsbehälter gefüllt mit einer Monomerflüssigkeit als zweite Ausgangskomponente lagert, umfassend die Verfahrensschritte:
a. Bereitstellen der Vorrichtung in der ersten Konfiguration;
b. Axiales Verschieben des Förderkolbens aus der ersten Position in die zweite Position unter fluidleitendem Öffnen des Monomerflüssigkeitsbehälters;
c. Fließen der Monomerflüssigkeit aus dem Reservoir in den Hohlzylinder;
d. Verbringen der Vorrichtung aus der ersten Konfiguration in die zweite Konfiguration;
e. Axiales Verschieben des Förderkolbens aus der zweiten Position in Richtung der dritten Position unter Fördern der Monomerflüssigkeit aus dem Hohlzylinder in den Innenraum;
f. Mischen des Knochenzementpulvers und der Monomerflüssigkeit in der Mischeinheit unter Bereitstellen des Knochenzementteigs.

Die Vorrichtung kann zu Beginn des Verfahrens bereits in der ersten Konfiguration vorliegen, beispielsweise kann die Vorrichtung bereits in der ersten Konfiguration vom Anwender käuflich erworben sein, oder der Anwender bringt die Vorrichtung zu Beginn des Verfahrens in die erste Konfiguration.

Falls die Vorrichtung eine Transportsicherung aufweist, sollte diese zu Beginn des Verfahrens entfernt werden.

Um den Monomerflüssigkeitsbehälter fluidleitend zu öffnen, wird in Verfahrensschritt b. der Förderkolben, während sich die Vorrichtung in der ersten Konfiguration befindet, aus der ersten Position, welche als Ausgangsposition des Förderkolbens angesehen werden kann, in die zweite Position verschoben. Da in der ersten Konfiguration der Förderkolben mit dem Öffnungsmittel, vorzugsweise über einen Mitnehmer, verbunden ist, führt das Verschieben des Förderkolbens zu einem Verschieben des Öffnungsmittels, welches den Monomerflüssigkeitsbehälter fluidleitend öffnet.

Nach dem Öffnen des Monomerflüssigkeitsbehälters kommt es in Verfahrensschritt c. zu einem, im Wesentlichen vollständigen, Fließen der Monomerflüssigkeit aus dem Reservoir in den Hohlzylinder der Transfereinheit.

Um die Monomerflüssigkeit aus der Transfereinheit, insbesondere dem Hohlzylinder der Transfereinheit, in den Innenraum der Mischeinheit zu fördern, wird die Vorrichtung aus der ersten Konfiguration in die zweite Konfiguration überführt.

Das eigentliche Fördern der Monomerflüssigkeit erfolgt in Verfahrensschritt e. durch axiales Verschieben des Förderkolbens aus der zweiten Position in Richtung der dritten Position. Je weiter das Verschieben in Richtung der dritten Position durchgeführt wird, desto mehr der im Hohlzylinder befindlichen Monomerflüssigkeit wird in die Mischeinheit gefördert. Je nach Wunsch des Anwenders kann das Verschieben daher bis in die dritte Position durchgeführt werden oder lediglich bis in eine Zwischenposition zwischen der zweiten Position und der dritten Position. Vorzugsweise weist die Vorrichtung, insbesondere der Förderkolben, ein lösbares Kontrollelement auf, welches dem Anwender ein passgenaues Verschieben des Förderkolbens in den Hohlzylinder erleichtern. Das Kontrollelement erlaubt ein Verschieben des Förderkolbens auf eine durch den Anwender festgelegte Zwischenposition zwischen der zweiten Position und der dritten Position, so dass eine vorbestimmte Menge der Monomerflüssigkeit in die Mischeinheit gefördert werden kann, während ein restlicher Teil der Monomerflüssigkeit in der Transfereinheit verbleibt und nicht zum Anmischen des Knochenzementteigs zur Verfügung steht.

Durch das Fördern der Monomerflüssigkeit in die Mischeinheit kommt es im Innenraum derselben zu einem Kontakt der Monomerflüssigkeit zum Knochenzementpulver. Unter Mischen dieser beiden Ausgangskomponenten kommt es zur Bereitstellung des Knochenzementteigs im Mischsystem. Das Mischen kann beispielsweise unter Verwendung eines Mischstabes, welcher reversibel im Innenraum axial verschoben wird, erfolgen.

Eine Ausführungsform des Verfahrens ist dadurch gekennzeichnet, dass das Verbringen der Vorrichtung aus der ersten Konfiguration in die zweite Konfiguration durch eine Drehbewegung geschieht. In einer Ausgestaltungsform erfolgt der Konfigurationswechsel durch ein Drehen des Förderkolbens. In einer weiteren Ausgestaltungsform erfolgt der Konfigurationswechsel durch ein Drehen eines Ringes. Beide Möglichkeiten sind in den vorhergehenden Ausführungsformen der Vorrichtung näher beschrieben.

Die Vorrichtung ist dadurch gekennzeichnet, dass diese einen Knochenzementteig aus zwei Ausgangskomponenten bereitstellt. Unter einem Knochenzementteig wird eine Substanz verstanden, die geeignet ist im Bereich der Medizintechnik eine stabile Verbindung zwischen künstlichen Gelenken, wie beispielsweise Hüft- und Kniegelenken, und Knochenmaterial zu erstellen. Durch Aushärtung wird aus einem Knochenzementteig ein Knochenzement. Bevorzugt handelt es sich bei diesen Knochenzementen um Polymethylmethacrylat-Knochenzemente (PMMA-Knochenzemente). PMMA-Knochenzemente kommen schon lange in medizinischen Anwendungen zum Einsatz und gehen auf Arbeiten von Sir Charnley zurück (vgl. Charnley, J. Anchorage of the femoral head prosthesis of the shaft of the femur. J. Bone Joint Surg. 1960; 42, 28-30.). PMMA-Knochenzemente können dabei aus einem Knochenzementpulver als erster Ausgangskomponente und einer Monomerflüssigkeit als zweiter Ausgangskomponente hergestellt werden. Bei geeigneter Zusammensetzung können die beiden Ausgangskomponenten getrennt voneinander lagerstabil sein. Bei Inkontaktbringen der beiden Ausgangskomponenten entsteht durch Quellung der Polymerbestandteile des Knochenzementpulvers ein plastisch verformbarer Knochenzementteig. Dabei wird eine Polymerisation des Monomers durch Radikale eingeleitet. Mit fortschreitender Polymerisation des Monomers erhöht sich die Viskosität des Knochenzementteigs, bis dieser vollständig aushärtet.

Unter einem Knochenzementpulver wird ein Pulver verstanden, welches mindestens ein partikuläres Polymethylmethacrylat und/oder ein partikuläres Polymethylmethacrylat-Copolymer umfasst. Beispiele für Copolymere sind Styren und/oder Methylacrylat. In einer Ausgestaltungsform kann das Knochenzementpulver zusätzlich ein hydrophiles Additiv umfassen, welches die Verteilung der Monomerflüssigkeit innerhalb des Knochenzementpulvers unterstützt. In einer weiteren Ausgestaltungsform kann das Knochenzementpulver zusätzlich einen Initiator, welcher die Polymerisation einleitet, umfassen. In einer weiteren Ausgestaltungsform kann das Knochenzementpulvers zusätzlich einen Röntgenopaker umfassen. In noch einer weiteren Ausgestaltungsform kann das Knochenzementpulver zusätzlich pharmazeutisch aktive Substanzen, wie beispielsweise Antibiotika, umfassen.

Bevorzugt umfasst das Knochenzementpulver als hydrophiles Additiv mindestens ein partikuläres Polymethylmethacrylat und/oder ein partikuläres Polymethylmethacrylat-Copolymer, einen Initiator und einen Röntgenopaker oder besteht aus diesen Komponenten. Weiter bevorzugt umfasst das Knochenzementpulver mindestens ein partikuläres Polymethylmethacrylat und/oder ein partikuläres Polymethylmethacrylat-Copolymer, einen Initiator, einen Röntgenopaker und ein hydrophiles Additiv oder besteht aus diesen Komponenten. Am bevorzugtesten umfasst das Knochenzementpulver mindestens ein partikuläres Polymethylmethacrylat und/oder ein partikuläres Polymethylmethacrylat-Copolymer, einen Initiator, einen Röntgenopaker, ein hydrophiles Additiv und ein Antibiotikum oder besteht aus diesen Komponenten.

Erfindungsgemäß kann die Partikelgröße des partikulären Polymethylmethacrylat und/oder des partikulären Polymethylmethacrylat-Copolymers des Knochenzementpulvers der Siebfraktion kleiner 150 µm, bevorzugt kleiner 100 µm, entsprechen.

Erfindungsgemäß kann das hydrophile Additiv partikulär und/oder faserförmig ausgestaltet sein. In einer weiteren Ausgestaltungsform kann das hydrophile Additiv schwerlöslich, bevorzugt unlöslich, in Methylmethacrylat sein. In einer weiteren Ausgestaltungsform kann das hydrophile Additiv ein Aufsaugvermögen von mindestens 0,6 g Methylmethacrylat pro Gramm hydrophiles Additiv besitzen. In einer weiteren Ausgestaltungsform kann das hydrophile Additiv eine chemische Substanz mit mindestens einer OH-Gruppe aufweisen. Dabei kann bevorzugt vorgesehen sein, dass das hydrophile Additiv kovalent gebundene OH-Gruppen an seiner Oberfläche besitzt. Beispiele für solche bevorzugten hydrophilen Additive können Additive ausgewählt aus der Gruppe umfassend Cellulose, Oxycellulose, Stärke, Titandioxid und Siliziumdioxid sein, wobei pyrogenes Siliziumdioxid besonders bevorzugt ist. In einer Ausgestaltungsform kann die Partikelgröße des hydrophilen Additivs der Siebfraktion kleiner 100 µm, bevorzugt kleiner 50 µm und am bevorzugtesten kleiner 10 µm entsprechen. Das hydrophile Additiv kann in einer Menge von 0,1 bis 2,5 Gew.-% bezogen auf das Gesamtgewicht des Knochenzementpulvers enthalten sein.

Erfindungsgemäß kann der Initiator Dibenzoylperoxid enthalten oder aus Dibenzoylperoxid bestehen.

Erfindungsgemäß versteht man unter einem Röntgenopaker eine Substanz, welche es erlaubt, den Knochenzement auf röntgendiagnostischen Aufnahmen sichtbar zu machen. Beispiele für Röntgenopaker können Bariumsulfat, Zirkondioxid und Kalziumcarbonat umfassen. Erfindungsgemäß kann die pharmazeutisch aktive Substanz ein oder mehrere Antibiotika und gegebenenfalls zugesetzte Co-Faktoren für das eine oder die mehreren Antibiotika umfassen. Bevorzugt besteht die pharmazeutisch aktive Substanz aus einem oder mehreren Antibiotika und gegebenenfalls zugesetzten Co-Faktoren für das eine oder die mehreren Antibiotika. Beispiele für Antibiotika sind unter anderem Gentamicin, Clindamycin und Vancomycin. Erfindungsgemäß kann die Monomerflüssigkeit das Monomer Methylmethacrylat umfassen oder aus Methylmethacrylat bestehen. In einer Ausgestaltungsform umfasst die Monomerflüssigkeit neben dem Monomer einen darin gelösten Aktivator, wie beispielsweise N,N-Dimethyl-p-toluidin, oder besteht aus Methylmethacrylat und N,N-Dimethyl-p-toluidin.

Die für die Vorrichtung offenbarten Merkmale sind auch für das Verfahren offenbart und umgekehrt.

### Figuren

Die Erfindung wird im Folgenden durch Figuren weiter beispielhaft illustriert. Die Erfindung ist nicht auf die Figuren beschränkt.

### Es zeigen

- Fig. 1: einen schematischen Längsschnitt einer beispielhaften Vorrichtung zum Bereitstellen eines Knochenzementteigs umfassend eine Mischeinheit, ein Reservoir mit einer Monomerflüssigkeit und eine Transfereinheit, wobei die Vorrichtung sich in einer ersten Konfiguration befindet und ein Förderkolben in einer ersten Position angeordnet ist,
- Fig. 2: die Vorrichtung aus Figur 1 in einer zweiten Position des Förderkolbens,
- Fig. 3: die Vorrichtung aus den Figuren 1 und 2 in einer zweiten Konfiguration und bei einem Fördern der Monomerflüssigkeit aus der Transfereinheit in die Mischeinheit,
- Fig. 4: die Vorrichtung aus den Figuren 1 bis 3 bei einem Mischen des Knochenzementpulvers und der Monomerflüssigkeit unter Bereitstellen des Knochenzementteigs,
- Fig. 5: einen schematischen Längsschnitt einer weiteren beispielhaften Vorrichtung zum Bereitstellen eines Knochenzementteigs umfassend eine Mischeinheit, ein Reservoir mit einer Monomerflüssigkeit und eine Transfereinheit, wobei die Vorrichtung sich in einer ersten Konfiguration befindet und ein Förderkolben in einer ersten Position angeordnet ist,
- Fig. 6: die Vorrichtung aus Figur 5 in einer zweiten Konfiguration,
- Fig. 7: die Vorrichtung aus den Figuren 5 und 6 in einer Zwischenposition des Förderkolbens, und
- Fig. 8: ein Flussdiagramm eines Verfahrens zum Bereitstellen eines Knochenzementteigs.

### Beschreibung der Figuren

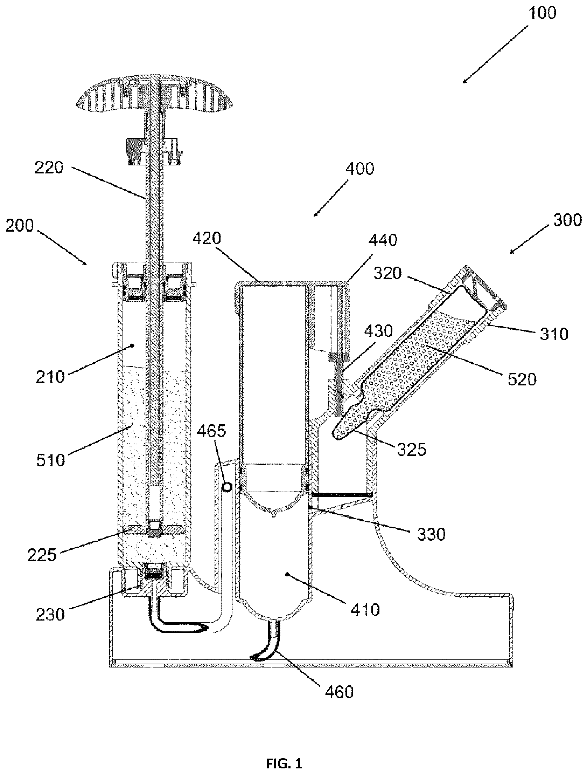

**Figur** 1 zeigt einen schematischen Längsschnitt einer beispielhaften Ausführung einer Vorrichtung 100 zum Bereitstellen eines Knochenzementteigs aus zwei Ausgangskomponenten in einem Ausgangszustand. Die Vorrichtung 100 umfasst eine Mischeinheit 200, ein Reservoir 300 und eine Transfereinheit 400, über welche die Mischeinheit 200 und das Reservoir 300 fluidleitend verbunden oder verbindbar ist.

Die Mischeinheit 200 dient einem Anmischen der Ausgangskomponenten des Knochenzementteigs und umfasst einen Innenraum 210, in dem ein Knochenzementpulver 510 als erste Ausgangskomponente des bereitzustellenden Knochenzementteigs lagert, sowie ein Mischelement 220 in Form eines Mischstabes. Das Mischelement 220 ist reversibel innerhalb des Innenraums 210 verschiebbar sowie drehbar um eine Längsachse gelagert, so dass Mischflügel 225 ein homogenes Anmischen der Ausgangskomponenten sicherstellen können. Die Mischeinheit 200 ist reversibel lösbar über ein Gewinde 230 mit dem restlichen Teil der Vorrichtung 100 verbunden, so dass nach einem Abtrennen der Mischeinheit 200 der in dieser bereitgestellte Knochenzementteig aus der Mischeinheit 200 an eine gewünschte Stelle applizierbar ist.

Das Reservoir 300 umfasst einen Reservoirbehälter 310, in dem ein fluidleitend geschlossener Monomerflüssigkeitsbehälter 320 in Form einer Ampulle, insbesondere einer Glasampulle, lagert. Innerhalb des Monomerflüssigkeitsbehälters 320 lagert eine Monomerflüssigkeit 520 als zweite Ausgangskomponente des Knochenzementteigs.

Räumlich zwischen der Mischeinheit 200 und dem Reservoir 300 ist die Transfereinheit 200 angeordnet. Die Transfereinheit 200 umfasst einen Hohlzylinder 410 und einen im Hohlzylinder 410 axial verschiebbaren Förderkolben, welche als ein Kolben-Zylinder-System zum Fördern der Monomerflüssigkeit 520 aus dem Hohlzylinder 410 in den Innenraum 210 zusammenwirken. Dazu ist der Hohlzylinder 410 zum einen fluidleitend über eine Verbindungsleitung 430 mit dem Innenraum 210 sowie, zumindest in der gezeigten Anordnung des Förderkolbens 420 innerhalb des Hohlzylinders 410, über eine Durchführung 330 fluidleitend mit dem Reservoir 300 verbunden. Die Verbindungsleitung 460 zwischen der Transfereinheit 400 und der Mischeinheit 200 ist in einer Schlaufe mit einem hohen Scheitelpunkt 465 angeordnet, um ein unkontrolliertes Durchfließen der Monomerflüssigkeit 520 durch die Verbindungsleitung 460 in die Mischeinheit 200, insbesondere den Innenraum 210, zu verhindern. Um ein möglichst vollständiges Fördern der Monomerflüssigkeit 520 aus dem Hohlzylinder 410 in den Innenraum 210 zu gewährleisten, sind der Hohlzylinder 410 und der Förderkolben 420 bombiert ausgeformt und das Verbindungselement 460 ist an der tiefsten Stelle des Hohlzylinders 410 angeordnet.

Zum fluidleitenden Öffnen des Monomerflüssigkeitsbehälters 320 weist die Vorrichtung 100 ein Öffnungsmittel 430 in Form eines Keils auf. Das Öffnungsmittel 430 ist axial verschiebbar über einem Ampullenkopf 325 des Monomerflüssigkeitsbehälters 320 gelagert, so dass ein axiales Verschieben des Öffnungsmittels 430 ein Abbrechen des Ampullenkopfes 325 bewirken kann und dadurch der Monomerflüssigkeitsbehälter 320 fluidleitend öffenbar ist. Um das Öffnungsmittel 430 derart zu verschieben, ist dieses in Figur 1 über einen Mitnehmer 440 mit dem Förderkolben 420 verbunden. Dies bedeutet, dass ein axiales Verschieben des Förderkolbens 420 innerhalb des Hohlzylinders 410 sich über den Mitnehmer 440 direkt auf das Öffnungsmittel 430 überträgt. Die Vorrichtung 100 ist in Figur 1 somit in einer ersten Konfiguration angeordnet, wobei sich der Förderkolben 420 in einer ersten Position, seiner Ausgangsposition, befindet.

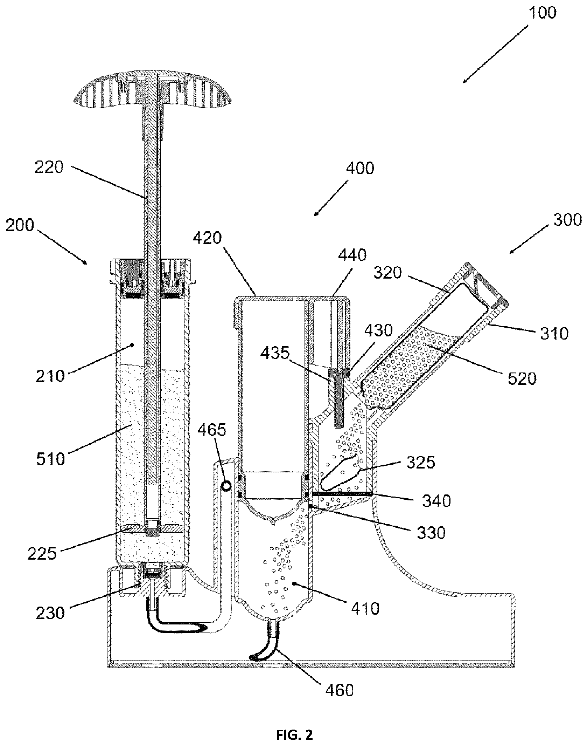

**Figur** 2 zeigt die Vorrichtung aus Figur 1, wobei sich die Vorrichtung 100 weiterhin in der ersten Konfiguration befindet, der Förderkolben 420 aber aus seiner in Figur 1 gezeigten ersten Position axial im Hohlzylinder 410 in eine zweite Position verschoben wurde. In der ersten Konfiguration überträgt sich, wie bereits beschrieben, eine Verschiebung des Förderkolbens 420 direkt über den Mitnehmer 440 auf das Öffnungsmittel 430. Durch das Verschieben des Förderkolbens 420 in die zweite Position wurde das Öffnungsmittel 430 gegen den Ampullenkopf 325 des Monomerflüssigkeitsbehälters 320 gepresst, welche daraufhin abgebrochen ist und damit den Monomerflüssigkeitsbehälter 320 fluidleitend geöffnet hat. Die Monomerflüssigkeit 520 fließt im gezeigten Zeitpunkt gerade aus dem fluidleitend geöffneten Monomerflüssigkeitsbehälter 320 durch die Durchführung 330 in den Hohlzylinder 410 der Transfereinheit 400. In der zweiten Position des Förderkolbens 420 liegt das Öffnungsmittel 430 an einem Anschlag 435 an. Der Anschlag 435 blockiert ein fortgeführtes axiales Verschieben des Öffnungsmittels 430 in der zum fluidleitenden Öffnen des Monomerflüssigkeitsbehälters 320 eingeschlagenen Richtung. In der ersten Konfiguration der Vorrichtung 100 ist somit auch ein fortgeführtes Verschieben des Förderkolbens 420 aus der zweiten Position in Richtung einer dritten Position unterbunden. Ein Verbleiben der Vorrichtung 100 in der ersten Konfiguration verhindert damit ein, insbesondere unbeabsichtigtes, Fördern der Monomerflüssigkeit 520 aus dem Hohlzylinder 410 in die Mischeinheit 200.

Um eine Kontamination des bereitzustellenden Knochenzementteigs mit Teilstücken, insbesondere mit Glassplittern, des fluidleitend geöffneten Monomerflüssigkeitsbehälters 320 zu unterbinden, befindet sich innerhalt des Reservoirs 300 ein Filterelement 340, welches die Teilstücke im Reservoir 300 zurückhält. In der Ausführungsform wird insbesondere der Ampullenkopf 325 innerhalb des Reservoirs 300 vom Filterelement zurückgehalten.

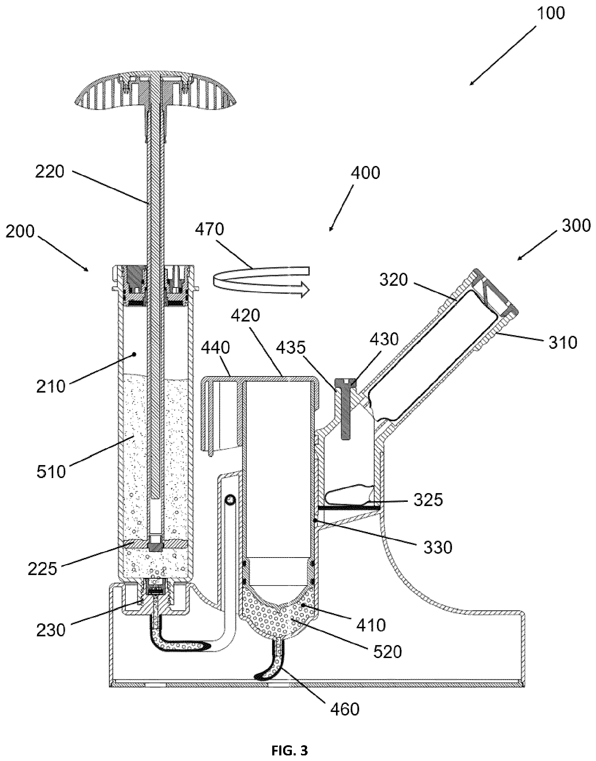

**Figur 3** zeigt die Vorrichtung 100 aus den Figuren 1 und 2, wobei sich die Vorrichtung 100 in einer zweiten Konfiguration befindet. In der zweiten Konfiguration ist der Förderkolben 420 nicht mehr über den Mitnehmer 440 mit dem Öffnungsmittel 430 verbunden, so dass der Förderkolben 420 auch nicht mehr über das am Anschlag 435 anliegende Öffnungsmittel 430 in seiner Bewegungsfreiheit innerhalb des Hohlzylinders 410 eingeschränkt ist. Die Vorrichtung 100 wurde aus der ersten Konfiguration in die zweite Konfiguration über eine Drehbewegung 470 (angedeutet durch Pfeil) des Förderkolbens 420 verbracht. Durch die Drehbewegung 470 steht der Mitnehmer 440 nicht mehr in Eingriff mit dem Öffnungsmittel 430. Der Förderkolben wurde im gezeigten Zeitpunkt aus seiner zweiten Position in Richtung einer dritten Position axial im Hohlzylinder 410 verschoben, was zum einen die Durchführung 330 fluidleitend geschlossen hat und zum anderen ein Fördern der Monomerflüssigkeit 520 aus der Transfereinheit 400 über die Verbindungsleitung 460 in die Mischeinheit 200 eingeleitet hat.

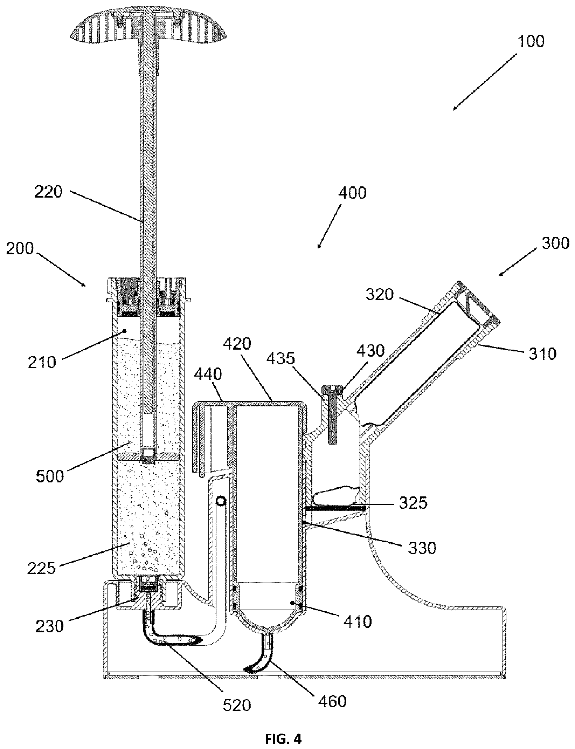

**Figur 4** zeigt die Vorrichtung 100 aus den Figuren 1 bis 3 beim Bereitstellen des Knochenzementteigs 500. Die Vorrichtung 100 befindet sich weiterhin, wie in Figur 3, in der zweiten Konfiguration, wobei der Förderkolben 420 bis in die dritte Position verschoben wurde. Dies hat zu einem im Wesentlichen vollständigen Fördern der Monomerflüssigkeit 520 (lediglich Reste in der Verbindungsleitung 460 übrig) in die Mischeinheit 200 geführt, wo die beiden Ausgangskomponenten durch wiederholtes Verschieben und Drehen des Mischelements 220 zum Knochenzementteig 500 angemischt wurden. Der Knochenzementteig 500 steht somit nach einem Abtrennen der Mischeinheit 200 von der restlichen Vorrichtung 100 zum Austragen an eine gewünschte Stelle bereit.

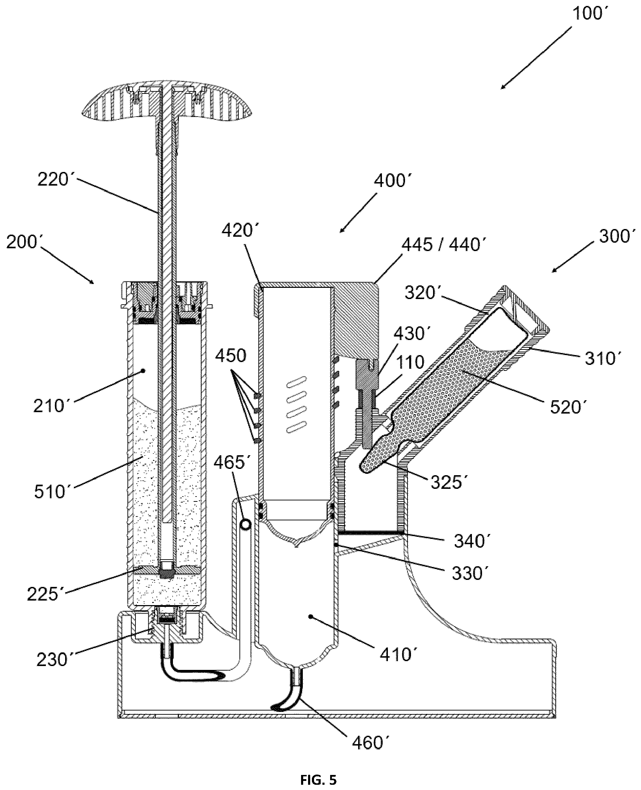

**Figur 5** zeigt einen schematischen Längsschnitt einer weiteren beispielhaften Ausführung einer Vorrichtung 100' zum Bereitstellen eines Knochenzementteigs aus zwei Ausgangskomponenten in einem Ausgangszustand.

Die Ausführungsform gemäß Figur 5 stimmt weitgehend mit der vorstehend beschriebenen und in den vorstehenden Figuren dargestellten Ausführungsform und deren Benutzung überein, so dass zur Vermeidung von Wiederholungen auf die vorstehende Beschreibung verwiesen wird. Eine Struktur, die aus der Beschreibung der vorstehenden Figuren wiederholt wird, weist dasselbe Bezugszeichen auf mit einem Apostroph auf.

Die Vorrichtung 100' befindet sich, analog zu Vorrichtung 100 in Figur 1, in einer ersten Konfiguration und der Förderkolben 420' ist in der ersten Position.

Die Vorrichtung 100' weist am Öffnungsmittel 430' eine lösbare Transportsicherung 110 auf, welche ein unbeabsichtigtes Verschieben des Öffnungsmittels 430' und somit fluidleitendes Öffnen des Monomerflüssigkeitsbehälters 320' verhindert. Weiterhin umfasst die Vorrichtung 100' im Gegensatz zu Vorrichtung 100 aus den Figuren 1 bis 4 mehrere, insbesondere 4, vom Förderkolben 420' lösbare Kontrollelemente 450. Die Kontrollelemente 450 erlauben es einem Anwender der Vorrichtung 100' den Förderkolben 420' lediglich auf eine durch das einzelne Kontrollelement 450 vorbestimmte Zwischenposition zwischen der zweiten Position und der dritten Position des Förderkolbens 420' im Hohlzylinder 410' zu verschieben und so die sich dort befindliche Monomerflüssigkeit 520' lediglich zu einem vorbestimmten Anteil in die Mischeinheit 200' zu fördern. In der gezeigten Ausführungsform der Vorrichtung 100' ist der Mitnehmer 440' an einem um den Förderkolben 420' drehbaren Ring, insbesondere einem kappenartigen Ring, angeordnet. Ein Verbringen der Vorrichtung 100' aus der ersten Konfiguration in eine zweite Konfiguration ist somit über ein Drehen des Ringes 445, und nicht wie bei Vorrichtung 100 aus den Figuren 1 bis 4 über ein Drehen des Förderkolbens 420 selbst, ermöglicht.

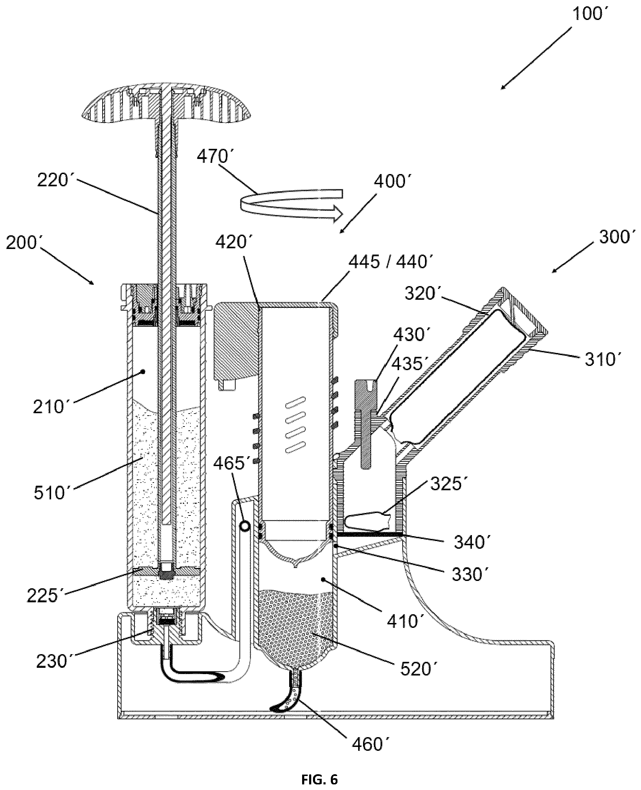

**Figur** 6 zeigt die Vorrichtung 100' aus Figur 5 nach einem fluidleitenden Öffnen des Monomerflüssigkeitsbehälters 320'. Dazu wurde die Transportsicherung 110 entfernt und der Förderkolben 420' aus der ersten Position in eine zweite Position innerhalb des Hohlzylinders 410' axial verschoben. Zum gezeigten Zeitpunkt befindet sich die Monomerflüssigkeit 520 bereits im Wesentlichen vollständig im Hohlzylinder 410' und der Förderkolben 420' wurde durch eine Drehbewegung 470' (angedeutet durch einen Pfeil) des Ringes 445 vom Öffnungsmittel 430 getrennt, was die Vorrichtung 100' aus der ersten Konfiguration in eine zweite Konfiguration überführt hat.

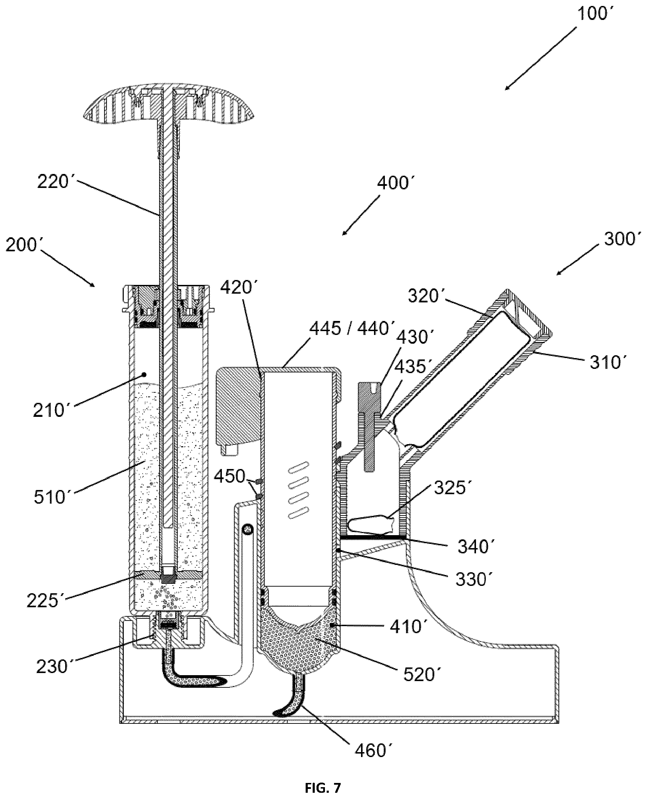

**Figur** 7 zeigt die Vorrichtung 100' aus den Figuren 5 und 6, wobei sich die Vorrichtung 100' weiterhin in der zweiten Konfiguration befindet und der Förderkolben 420' aus der zweiten Position in Figur 6 auf eine Zwischenposition zwischen der zweiten Position und einer dritten Position verschoben wurde. Die Zwischenposition wird in der gezeigten Ausführungsform durch das Vorhandenseins eines der Kontrollelemente 450 bestimmt, welches ein fortgesetztes Einschieben des Förderkolbens 420' in den Hohlzylinder 410' in die dritte Position unterbindet. Dazu wurden die zwei unteren Kontrollelemente 450 (vgl. Figur 5 oder 6) entfernet und das vormals dritte Kontrollelement von unten definiert die Zwischenposition. Dies ermöglicht ein vorbestimmtes Fördern lediglich eines Teils der Monomerflüssigkeit 520' aus der Transfereinheit 200' in die Mischeinheit' während der restliche Teil der Monomerflüssigkeit 520', zumindest bis zu einem Entfernen der restlichen Kontrollelement 450 vom Förderkolben 420' und einem fortgeführten verschieben des Förderkolbens 420' in die dritte Position, im Hohlzylinder 410' verbleibt. So kann ein bestimmtes Mischungsverhältnis der Ausgangskomponenten im Knochenzementteig erreicht werden.

Ein Anmischen und Bereitstellen des Knochenzementteigs kann analog zur Ausführungsform der Vorrichtung 100 der Figuren 1 bis 4 erreicht werden.

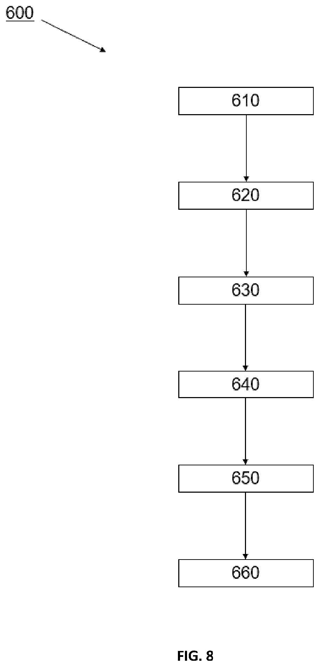

**Figur** 8 zeigt ein Verfahren 600 zur Bereitstellung eines Knochenzementteigs 520 aus zwei Ausgangskomponenten mittels den vorstehend beschriebenen Vorrichtung 100, 100' aus den Figuren 1 bis 4 und 5 bis 7 umfassend die Verfahrensschritte 610 bis 660.

Im Innenraum 210, 210' der Mischeinheit 200, 200' lagert ein Knochenzementpulver 510, 510' als erste Ausgangskomponente und im Reservoirbehälter 310, 310' des Reservoirs 300, 300' lagert ein mit einer Monomerflüssigkeit 520, 520' als zweite Ausgangskomponente befüllter Monomerflüssigkeitsbehälter 320, 320'.

In einem Verfahrensschritt 610 wird die Vorrichtung 100, 100' in der ersten Konfiguration bereitgestellt. Die erste Konfiguration kann beispielsweise durch einen Anwender der Vorrichtung 100, 100' installiert werden oder der Anwender der Vorrichtung 100, 100' hat kann diese beispielsweise bereits in der ersten Konfiguration erworben erhaben.

Sollte die Vorrichtung 100, 100' eine Transportsicherung 110 aufweisen, so ist diese vorzugsweise vor den darauffolgenden Verfahrensschritten zu entfernen.

In einem Verfahrensschritt 620 erfolgt ein axiales Verschieben des Förderkolbens 420, 420' aus der ersten Position, welche vorzugsweise die Ausgangsposition des Förderkolbens 420, 420' ist, in die zweite Position unter fluidleitendem Öffnen des Monomerflüssigkeitsbehälters 320, 320'. Zur Klarstellung sei noch einmal darauf hingewiesen, dass sich die Vorrichtung 100, 100' für diesen Verfahrensschritt in der ersten Konfiguration befindet, so dass sich das Verschieben des Förderkolbens 420, 420' auf das Öffnungsmittel 430, 430' überträgt. Vorzugsweise erfolgt das Verschieben des Förderkolbens 420, 420' durch eine Kraftausübung des Anwenders der Vorrichtung 100, 100' auf den Förderkolben 420, 420'. Alternativ oder ergänzend kann das Verschieben des Förderkolbens 420, 420' auch durch einen an der Vorrichtung 100, 100', insbesondere am Innenraum 210, 210' angelegten Unterdruck ausgelöst werden. Dies gilt auch für jedes weitere Verschieben des Förderkolbens 420, 420' im Zuge des Verfahrens 600.

In einem Verfahrensschritt 630 fließt die Monomerflüssigkeit 520, 520' aus dem Reservoir 300, 300' in den Hohlzylinder 410, 410'. Vorzugsweise erfolgt das Fließen der Monomerflüssigkeit 520, 520' gemäß der Schwerkraft. Die Vorrichtung 100, 100' ist dazu entsprechend räumlich auszurichten.

In einem Verfahrensschritt 640 wird die Vorrichtung 100, 100' aus der ersten Konfiguration in die zweite Konfiguration überführt. Vorzugsweise geschieht durch eine Drehbewegung 470, 470', vorzugsweise des Förderkolbens 420 oder des Ringes 445.

In einem Verfahrensschritt 650 erfolgt ein axiales Verschieben des Förderkolbens 420, 420' aus der zweiten Position in Richtung der dritten Position unter Fördern der Monomerflüssigkeit 520, 520' aus dem Hohlzylinder 410, 410' in den Innenraum 210, 210'. Je weiter der Förderkolben 420, 420' in Richtung der dritten Position verschoben wird, desto mehr der im Hohlzylinder 410, 410' befindlichen Monomerflüssigkeit 520, 520' wird dabei in den Innenraum 210, 210' gefördert, bis bei einem Erreichen der dritten Position die Monomerflüssigkeit 520, 520' im Wesentlichen vollständig gefördert würde oder wird. Die Menge an geförderter Monomerflüssigkeit 520, 520' kann so vom Anwender der Vorrichtung 100, 100' selbst bestimmt werden. Dabei kann der Anwender durch die Verwendung von Kontrollelementen 450 unterstützt werden.

In einem Verfahrensschritt 660 kommt es zu einem Mischen des Knochenzementpulvers 510, 510' und der Monomerflüssigkeit 520, 520' unter Bereitstellen des Knochenzementteigs 500.

Anschließend kann es zu einem Austragen des Knochenzementteigs 500 aus der Mischeinheit 200, 200' an die vom Anwender gewünschte Stelle kommen. Dazu kann die Mischeinheit 200, 200' vorzugsweise von der restlichen Vorrichtung 100, 100' abgetrennt werden und mittels einer Austragsvorrichtung aus dem Innenraum 210, 210', vorzugsweise mittels einem im Inneren des Innenraums 210, 210' verschiebbaren Austragskolbens, ausgetragen werden.

### Bezugszeichen

- 100, 100': Vorrichtung
- 110: Transportsicherung
- 200, 200': Mischeinheit
- 210, 210': Innenraum
- 220, 220': Mischelement
- 225, 225': Mischflügel
- 230, 230': Gewinde
- 300, 300': Reservoir
- 310, 310': Reservoirbehälter
- 320, 320': Monomerflüssigkeitsbehälter
- 325, 325': Ampullenkopf
- 330, 330': Durchführung
- 340, 340': Filterelement
- 350: Transportsicherung
- 400, 400': Transfereinheit
- 410, 410': Hohlzylinder
- 420, 420': Förderkolben
- 430, 430': Öffnungsmittel
- 435, 435': Anschlag
- 440, 440': Mitnehmer
- 445: Ring
- 450: Kontrollelement
- 460, 460': Verbindungsleitung
- 465, 465': Scheitelpunkt
- 470, 470': Drehbewegung
- 500: Knochenzementteig
- 510, 510': Knochenzementpulver
- 520, 520': Monomerflüssigkeit
- 600: Verfahren
- 610: Bereitstellen
- 620: Verschieben
- 630: Fließen
- 640: Verbringen
- 650: Verschieben
- 660: Mischen

## Patentansprüche

1. Vorrichtung (100, 100') zum Bereitstellen eines Knochenzementteigs (500) aus zwei Ausgangskomponenten, umfassend
eine Mischeinheit (200, 200') mit einem Innenraum (210, 210'), in welchem ein Knochenzementpulver (510, 510') als erste Ausgangskomponente lagert oder lagerbar ist,
ein Reservoir (300, 300') mit einem Reservoirbehälter (310, 310'), in dem mindestens ein fluidleitend geschlossener Monomerflüssigkeitsbehälter (320, 320') gefüllt mit einer Monomerflüssigkeit (520, 520') als zweite Ausgangskomponente lagert oder lagerbar ist, eine Transfereinheit (400, 400') umfassend einen Hohlzylinder (410, 410') und einen axial im Hohlzylinder (410, 410') verschiebbaren Förderkolben (420, 420'),
wobei die Mischeinheit (200, 200') und das Reservoir (300, 300') über den Hohlzylinder (410, 410') fluidleitend miteinander verbunden oder verbindbar sind,
wobei der Monomerflüssigkeitsbehälter (320, 320') in der Vorrichtung (100, 100') derart zu öffnen ist, dass die Monomerflüssigkeit (520, 520') aus dem Reservoir (300, 300') in den Hohlzylinder (410, 410') über eine Durchführung (330, 330') fließbar ist und aus dem Hohlzylinder (410, 410') in den Innenraum (210, 210') durch ein axiales Verschieben des Förderkolbens (420, 420') im Hohlzylinder (410, 410') förderbar ist,
**dadurch gekennzeichnet, dass**
in einer ersten Konfiguration der Vorrichtung (100, 100') der Förderkolben (420, 420') mit einem Öffnungsmittel (430, 430') verbunden ist, so dass ein axiales Verschieben des Förderkolbens (420, 420') aus einer ersten Position in eine zweite Position das Öffnungsmittel (430, 430') gegen den Monomerflüssigkeitsbehälter (320, 320') schiebt, so dass der Monomerflüssigkeitsbehälter (320, 320') durch das Öffnungsmittel (430, 430') fluidleitend geöffnet werden kann und die Monomerflüssigkeit (520, 520') aus dem Reservoir (300, 300') über die Durchführung (330, 330') in den Hohlzylinder (410, 410') fließen kann, und dass
in einer zweiten Konfiguration der Vorrichtung (100, 100') der Förderkolben (420, 420') getrennt vom Öffnungsmittel (430, 430') ist, so dass die Monomerflüssigkeit (520, 520') durch ein fortgeführtes axiales Verschieben des Förderkolbens (420, 420') aus der zweiten Position in Richtung einer dritten Position aus dem Hohlzylinder (410, 410') in den Innenraum (210, 210') förderbar ist.

2. Vorrichtung (100, 100') nach Anspruch 1, wobei in der ersten Konfiguration der Förderkolben (420, 420') lediglich zwischen der ersten Position und zweiten Position verschiebbar ist.

3. Vorrichtung (100, 100') nach Anspruch 2, wobei in der ersten Konfiguration das Öffnungsmittel (430, 430') in der zweiten Position des Förderkolbens (420, 420') an einem Anschlag (435, 435') anliegt, so dass das fortgeführte axiale Verschieben aus der zweiten Position in Richtung der dritten Position unterbunden ist.

4. Vorrichtung (100, 100') nach einem der vorhergehenden Ansprüche, wobei das Öffnungsmittel (430, 430') ein Keil ist.

5. Vorrichtung (100, 100') nach Anspruch 4, wobei der Keil mindestens eine Durchbrechung aufweist, so dass die Monomerflüssigkeit (520, 520') durch die Durchbrechung und die Durchführung (330, 330') aus dem Reservoir (300, 300') in den Hohlzylinder (410, 410') fließbar ist.

6. Vorrichtung (100, 100') nach einem der vorhergehenden Ansprüche, wobei der Monomerflüssigkeitsbehälter (320, 320') eine Ampulle ist und im Reservoirbehälter (310, 310') lagert, wobei das Öffnungsmittel (430, 430') axial über einem Ampullenkopf (325, 325') der Ampulle angeordnet ist, so dass beim Verschieben des Förderkolbens (420, 420') aus der ersten Position in die zweite Position in der ersten Konfiguration der Vorrichtung (100, 100') das Öffnungsmittel (430, 430') gegen den Ampullenkopf (325, 325') pressbar und so die Ampulle fluidleitend öffenbar ist.

7. Vorrichtung (100, 100') nach einem der vorhergehenden Ansprüche, wobei der Förderkolben (420, 420') und das Öffnungsmittel (430, 430') in der ersten Konfiguration über einen Mitnehmer (440, 440') miteinander verbunden sind.

8. Vorrichtung (100, 100') nach Anspruch 7, wobei der Mitnehmer (440, 440') beim Verbringen der Vorrichtung (100, 100') aus der ersten Konfiguration in die zweite Konfiguration vom Öffnungsmittel (430, 430') getrennt wird.

9. Vorrichtung (100) nach Anspruch 8, wobei das Verbringen der Vorrichtung (100) aus der ersten Konfiguration in die zweite Konfiguration durch ein Drehen des Förderkolbens (420) um eine Förderkolbenlängsachse erreichbar ist.

10. Vorrichtung (100') nach Anspruch 8, wobei der Mitnehmer (440') an einem Ring (445) um den Förderkolben (420') angeordnet ist, so dass das Verbringen der Vorrichtung (100') aus der ersten Konfiguration in die zweite Konfiguration durch ein Drehen des Ringes (445) um den Förderkolben (420') erreichbar ist.

11. Vorrichtung (100') nach einem der vorhergehenden Ansprüche, wobei die Vorrichtung (100') eine Transportsicherung (110) aufweist, welches ein unbeabsichtigtes Verschieben des Öffnungsmittels (430') verhindert.

12. Vorrichtung (100') nach einem der vorhergehenden Ansprüche, wobei der Förderkolben (420') mindestens ein lösbares Kontrollelement (450) aufweist, welches das Verschieben des Förderkolbens (420') zwischen der zweiten Position und der dritten Position auf eine Zwischenposition begrenzt.

13. Vorrichtung (100, 100') nach einem der vorhergehenden Ansprüche, wobei der Förderkolben (420, 420') beim axialen Verschieben aus der zweiten Position in Richtung der dritten Position die Durchführung (330, 330') fluidleitend verschließt.

14. Verfahren (600) zur Bereitstellung eines Knochenzementteigs (500) aus zwei Ausgangskomponenten mittels einer Vorrichtung (100, 100') nach einem der Ansprüche 1 bis 13, wobei im Innenraum (210, 210') ein Knochenzementpulver (510, 510') als erste Ausgangskomponente und im Reservoirbehälter (310, 310') ein fluidleitend geschlossener Monomerflüssigkeitsbehälter (320, 320') gefüllt mit einer Monomerflüssigkeit (520, 520') als zweite Ausgangskomponente lagert, umfassend die Verfahrensschritte:
a. Bereitstellen (610) der Vorrichtung (100, 100') in der ersten Konfiguration;
b. Axiales Verschieben (620) des Förderkolbens (420, 420') aus der ersten Position in die zweite Position unter fluidleitendem Öffnen des Monomerflüssigkeitsbehälters (320, 320');
c. Fließen (630) der Monomerflüssigkeit (520, 520') aus dem Reservoir (300, 300') in den Hohlzylinder (410, 410');
d. Verbringen (640) der Vorrichtung (100, 100') aus der ersten Konfiguration in die zweite Konfiguration;
e. Axiales Verschieben (650) des Förderkolbens (420, 420') aus der zweiten Position in Richtung der dritten Position unter Fördern der Monomerflüssigkeit (520, 520') aus dem Hohlzylinder (410, 410') in den Innenraum (210, 210');
f. Mischen (660) des Knochenzementpulvers (510, 510') und der Monomerflüssigkeit (520, 520') in der Mischeinheit (200, 200') unter Bereitstellen des Knochenzementteigs (500).

15. Verfahren (600) nach Anspruch 14, wobei das Verbringen (640) der Vorrichtung (100, 100') aus der ersten Konfiguration in die zweite Konfiguration durch eine Drehbewegung (470, 470') geschieht.

## Claims

1. A device (100, 100') for preparing a bone cement paste (500) from two starting components, comprising
a mixing unit (200, 200') with an interior space (210, 210') in which a bone cement powder (510, 510') as the first starting component is stored or can be stored,
a reservoir (300, 300') with a reservoir container (310, 310') in which at least one fluid-conductingly closed monomer liquid container (320, 320') filled with a monomer liquid (520, 520') as the second starting component is stored or can be stored, a transfer unit (400, 400') comprising a hollow cylinder (410, 410') and a conveying piston (420, 420') which is axially displaceable in the hollow cylinder (410, 410'),
wherein the mixing unit (200, 200') and the reservoir (300, 300') are fluid-conductingly connected or connectable to each other via the hollow cylinder (410, 410'),
wherein the monomer liquid container (320, 320') in the device (100, 100') can be opened such that the monomer liquid (520, 520') can flow from the reservoir (300, 300') into the hollow cylinder (410, 410') via a passage (330, 330') and can be conveyed from the hollow cylinder (410, 410') into the interior space (210, 210') by an axial displacement of the conveying piston (420, 420') in the hollow cylinder (410, 410'),
**characterized in that**
in a first configuration of the device (100, 100'), the conveying piston (420, 420') is connected to an opening means (430, 430') so that an axial displacement of the conveying piston (420, 420') from a first position to a second position pushes the opening means (430, 430') against the monomer liquid container (320, 320') so that the monomer liquid container (320, 320') can be fluid-conductingly opened by the opening means (430, 430') and the monomer liquid (520, 520') can flow from the reservoir (300, 300') through the passage (330, 330') into the hollow cylinder (410, 410'), **and in that**
in a second configuration of the device (100, 100'), the conveying piston (420, 420') is separated from the opening means (430, 430') so that the monomer liquid (520, 520') can be conveyed from the hollow cylinder (410, 410') into the interior space (210, 210') by a continued axial displacement of the conveying piston (420, 420') from the second position toward a third position.

2. The device (100, 100') according to claim 1, wherein, in the first configuration, the conveying piston (420, 420') is only displaceable between the first position and the second position.

3. The device (100, 100') according to claim 2, wherein, in the first configuration, the opening means (430, 430') rests against a stop (435, 435') in the second position of the conveying piston (420, 420') so that the continued axial displacement from the second position toward the third position is prevented.

4. The device (100, 100') according to any of the preceding claims, wherein the opening means (430, 430') is a wedge.

5. The device (100, 100') according to claim 4, wherein the wedge comprises at least one through hole so that the monomer liquid (520, 520') can flow through the through hole and the passage (330, 330') from the reservoir (300, 300') into the hollow cylinder (410, 410').

6. The device (100, 100') according to any of the preceding claims, wherein the monomer liquid container (320, 320') is an ampoule and is stored in the reservoir container (310, 310'), wherein the opening means (430, 430') is arranged axially above an ampoule head (325, 325') of the ampoule so that, when the conveying piston (420, 420') is displaced from the first position to the second position in the first configuration of the device (100, 100'), the opening means (430, 430') can be pressed against the ampoule head (325, 325') and thus the ampoule can be fluid-conductingly opened.

7. The device (100, 100') according to any of the preceding claims, wherein the conveying piston (420, 420') and the opening means (430, 430') are connected to each other via a driver (440, 440') in the first configuration.

8. The device (100, 100') according to claim 7, wherein the driver (440, 440') is separated from the opening means (430, 430') when the device (100, 100') is moved from the first configuration to the second configuration.

9. The device (100) according to claim 8, wherein the movement of the device (100) from the first configuration to the second configuration can be achieved by rotation of the conveying piston (420) about a conveying piston longitudinal axis.

10. The device (100') according to claim 8, wherein the driver (440') is arranged on a ring (445) around the conveying piston (420') so that the movement of the device (100') from the first configuration to the second configuration can be achieved by rotation of the ring (445) about the conveying piston (420').

11. The device (100') according to any of the preceding claims, wherein the device (100') comprises a transport lock (110) which prevents unintentional displacement of the opening means (430').

12. The device (100') according to any of the preceding claims, wherein the conveying piston (420') comprises at least one releasable control element (450) which limits the displacement of the conveying piston (420') between the second position and the third position to an intermediate position.

13. The device (100, 100') according to any of the preceding claims, wherein the conveying piston (420, 420') fluid-conductingly closes the passage (330, 330') during the axial displacement from the second position toward the third position.

14. A method (600) for preparing a bone cement paste (500) from two starting components by means of a device (100, 100') according to any of claims 1 to 13, wherein a bone cement powder (510, 510') as the first starting component is stored in the interior space (210, 210') and a fluid-conductingly closed monomer liquid container (320, 320') filled with a monomer liquid (520, 520') as the second starting component is stored in the reservoir container (310, 310'), comprising the method steps of:
a. providing (610) the device (100, 100') in the first configuration;
b. axially displacing (620) the conveying piston (420, 420') from the first position to the second position with fluid-conducting opening of the monomer liquid container (320, 320');
c. the monomer liquid (520, 520') flowing (630) from the reservoir (300, 300') into the hollow cylinder (410, 410');
d. moving (640) the device (100, 100') from the first configuration to the second configuration;
e. axially displacing (650) the conveying piston (420, 420') from the second position toward the third position with the monomer liquid (520, 520') being conveyed from the hollow cylinder (410, 410') into the interior space (210, 210');
f. mixing (660) the bone cement powder (510, 510') and the monomer liquid (520, 520') in the mixing unit (200, 200') to provide the bone cement paste (500).

15. The method (600) according to claim 14, wherein the movement (640) of the device (100, 100') from the first configuration to the second configuration occurs by a rotary movement (470, 470').

## Revendications

1. Dispositif (100, 100') permettant de fournir une pâte de ciment osseux (500) à partir de deux composants de départ, comprenant
une unité de mélange (200, 200') comportant un espace intérieur (210, 210') dans lequel est stockée ou peut être stockée une poudre de ciment osseux (510, 510') en tant que premier composant de départ,
un réservoir (300, 300') comportant un récipient de réservoir (310, 310') dans lequel est stocké ou peut être stocké au moins un récipient pour liquide monomère (320, 320') fermé d'une manière conductrice de fluide et rempli d'un liquide monomère (520, 520') en tant que second composant de départ, une unité de transfert (400, 400') comprenant un cylindre creux (410, 410') et un piston de transport (420, 420') pouvant coulisser axialement dans le cylindre creux (410, 410'), dans lequel l'unité de mélange (200, 200') et le réservoir (300, 300') sont reliés ou peuvent être reliés l'un à l'autre d'une manière conductrice de fluide par l'intermédiaire du cylindre creux (410, 410'), dans lequel le récipient pour liquide monomère (320, 320') peut être ouvert dans le dispositif (100, 100') de telle sorte que le liquide monomère (520, 520') peut s'écouler depuis le réservoir (300, 300') dans le cylindre creux (410, 410') par l'intermédiaire d'un passage (330, 330') et peut être transporté hors du cylindre creux (410, 410') dans l'espace intérieur (210, 210') par un coulissement axial du piston de transport (420, 420') dans le cylindre creux (410, 410'),
**caractérisé en ce que**
dans une première configuration du dispositif (100, 100'), le piston de transport (420, 420') est relié à un moyen d'ouverture (430, 430'), de sorte qu'un coulissement axial du piston de transport (420, 420') d'une première position vers une deuxième position pousse le moyen d'ouverture (430, 430') contre le récipient pour liquide monomère (320, 320'), de sorte que le récipient pour liquide monomère (320, 320') peut être ouvert par le moyen d'ouverture (430, 430') d'une manière conductrice de fluide et le liquide monomère (520, 520') peut s'écouler hors du réservoir (300, 300') dans le cylindre creux (410, 410') par l'intermédiaire du passage (330, 330'), **et en ce que**
dans une seconde configuration du dispositif (100, 100'), le piston de transport (420, 420') est séparé du moyen d'ouverture (430, 430'), de sorte que le liquide monomère (520, 520') peut être transporté hors du cylindre creux (410, 410') dans l'espace intérieur (210, 210') par la poursuite d'un coulissement axial du piston de transport (420, 420') depuis la deuxième position vers une troisième position.

2. Dispositif (100, 100') selon la revendication 1, dans lequel, dans la première configuration, le piston de transport (420, 420') peut coulisser uniquement entre la première position et la deuxième position.

3. Dispositif (100, 100') selon la revendication 2, dans lequel, dans la première configuration, le moyen d'ouverture (430, 430') repose contre une butée (435, 435') dans la deuxième position du piston de transport (420, 420'), de sorte que la poursuite du coulissement axial de la deuxième position vers la troisième position est empêchée.

4. Dispositif (100, 100') selon l'une des revendications précédentes, dans lequel le moyen d'ouverture (430, 430') est une cale.

5. Dispositif (100, 100') selon la revendication 4, dans lequel la cale présente au moins une perforation, de sorte que le liquide monomère (520, 520') peut s'écouler hors du réservoir (300, 300') dans le cylindre creux (410, 410') à travers la perforation et le passage (330, 330').

6. Dispositif (100, 100') selon l'une des revendications précédentes, dans lequel le récipient pour liquide monomère (320, 320') est une ampoule et est stocké dans le récipient de réservoir (310, 310'), dans lequel le moyen d'ouverture (430, 430') est disposé axialement au-dessus d'une tête d'ampoule (325, 325') de l'ampoule, de sorte que, lorsque le piston de transport (420, 420') coulisse de la première position vers la deuxième position dans la première configuration du dispositif (100, 100'), le moyen d'ouverture (430, 430') peut être pressé contre la tête d'ampoule (325, 325') et l'ampoule peut ainsi être ouverte d'une manière conductrice de fluide.

7. Dispositif (100, 100') selon l'une des revendications précédentes, dans lequel le piston de transport (420, 420') et le moyen d'ouverture (430, 430') sont reliés l'un à l'autre dans la première configuration par l'intermédiaire d'un moyen d'entraînement (440, 440').

8. Dispositif (100, 100') selon la revendication 7, dans lequel le moyen d'entraînement (440, 440') est séparé du moyen d'ouverture (430, 430') lors du déplacement du dispositif (100, 100') de la première configuration vers la seconde configuration.

9. Dispositif (100) selon la revendication 8, dans lequel le déplacement du dispositif (100) de la première configuration vers la seconde configuration peut être obtenu par une rotation du piston de transport (420) autour d'un axe longitudinal de piston de transport.

10. Dispositif (100') selon la revendication 8, dans lequel le moyen d'entraînement (440') est disposé sur une bague (445) autour du piston de transport (420'), de sorte que le déplacement du dispositif (100') de la première configuration vers la seconde configuration peut être obtenu par une rotation de la bague (445) autour du piston de transport (420').

11. Dispositif (100') selon l'une des revendications précédentes, dans lequel le dispositif (100') présente un appareil de blocage de convoyage (110) qui empêche un coulissement involontaire du moyen d'ouverture (430').

12. Dispositif (100') selon l'une des revendications précédentes, dans lequel le piston de transport (420') présente au moins un élément de commande (450) amovible qui limite le coulissement du piston de transport (420') entre la deuxième position et la troisième position à une position intermédiaire.

13. Dispositif (100, 100') selon l'une des revendications précédentes, dans lequel le piston de transport (420, 420'), lors de son coulissement axial de la deuxième position vers la troisième position, obture le passage (330, 330') d'une manière conductrice de fluide.

14. Procédé (600) pour la fourniture d'une pâte de ciment osseux (500) à partir de deux composants de départ au moyen d'un dispositif (100, 100') selon l'une des revendications 1 à 13, dans lequel une poudre de ciment osseux (510, 510') est stockée dans l'espace intérieur (210, 210') en tant que premier composant de départ et un récipient pour liquide monomère (320, 320') fermé d'une manière conductrice de fluide et rempli d'un liquide monomère (520, 520') est stocké dans le récipient de réservoir (310, 310') en tant que second composant de départ, comprenant les étapes de procédé consistant à :
a. fournir (610) le dispositif (100, 100') dans la première configuration ;
b. faire coulisser axialement (620) le piston de transport (420, 420') de la première position vers la deuxième position pour ouvrir d'une manière conductrice de fluide le récipient pour liquide monomère (320, 320') ;
c. laisser s'écouler (630) le liquide monomère (520, 520') hors du réservoir (300, 300') dans le cylindre creux (410, 410') ;
d. déplacer (640) le dispositif (100, 100') de la première configuration vers la seconde configuration ;
e. faire coulisser axialement (650) le piston de transport (420, 420') de la deuxième position vers la troisième position pour transporter le liquide monomère (520, 520') hors du cylindre creux (410, 410') dans l'espace intérieur (210, 210') ;
f. mélanger (660) la poudre de ciment osseux (510, 510') et le liquide monomère (520, 520') dans l'unité de mélange (200, 200') pour fournir la pâte de ciment osseux (500).

15. Procédé (600) selon la revendication 14, dans lequel le déplacement (640) du dispositif (100, 100') de la première configuration vers la seconde configuration est effectué par un mouvement de rotation (470, 470').
